# EUROPÄISCHE PATENTSCHRIFT

(11) **EP 2 649 054 B1**
(45) Veröffentlichungstag und Bekanntmachung des Hinweises auf die Patenterteilung: **12.07.2017**
(21) Anmeldenummer: 11793742.5
(22) Anmeldetag: 05.12.2011
(51) Int. Cl.: C07D 265/16, C08G 73/02, C10L 1/233, C10L 1/2383, C10L 10/18, C10M 133/48, C10M 149/22

(54) **POLYTETRAHYDROBENZOXAZINE UND BISTETRAHYDROBENZOXAZINE UND IHRE VERWENDUNG ALS KRAFTSTOFFADDITIV ODER SCHMIERSTOFFADDITIV**
POLYTETRAHYDROBENZOXAZINES AND BISTETRAHYDROBENZOXAZINES AND THEIR USE AS FUEL ADDITIVES OR LUBRICANT ADDITIVE
POLYTETRAHYDROBENZOXAZINES ET BISTETRAHYDROBENZOXAZINES ET LEUR UTILISATION EN TANT QU'ADDITIF DE CARBURANT OU ADDITIF DE LUBRIFIANT

(30) Priorität: 09.12.2010 EP 10194307
(43) Veröffentlichungstag der Anmeldung: 16.10.2013
(73) Patentinhaber: BASF SE, 67056 Ludwigshafen am Rhein (DE)
(72) Erfinder: LANGE, Arno, 67098 Bad Dürkheim (DE); BÖHNKE, Harald, 68167 Mannheim (DE); GRABARSE, Wolfgang, 68163 Mannheim (DE); KÖNIG, Hannah, Maria, 68161 Mannheim (DE); HANSCH, Markus, 67346 Speyer (DE); VÖLKEL, Ludwig, 67117 Limburgerhof (DE); GARCIA CASTRO, Ivette, 67067 Ludwigshafen (DE)
(74) Vertreter: BASF IP Association
(86) Internationale Anmeldenummer: PCT/EP2011/071683
(87) Internationale Veröffentlichungsnummer: WO 2012/076428

(56) Entgegenhaltungen:
- WO-A1-01/25293
- WO-A1-2009/040584
- WO-A2-2007/099048
- US-A- 2 826 575
- US-A- 2 826 576
- US-A- 3 126 290
- DATABASE CA [Online] CHEMICAL ABSTRACTS SERVICE, COLUMBUS, OHIO, US; 2007, LU, ZAIJUN ET AL: "Process for preparation of benzoxazines and intermediates containing active functional groups", XP002667132, gefunden im STN Database accession no. 2007:1089419 -& CN 101 041 644 A (SHANDONG UNIVERSITY, PEOP. REP. CHINA) 26. September 2007 (2007-09-26)
- MANIKANNAN, RAMAIYAN ET AL: "Synthesis and biological activity of 6-alkyl/chloro-3-{4-(6-alkyl/chloro-2H-ben zo[e][1,3]oxazin-3(4H)- yl)phenyl}-3,4-dihydro-2H-benzo[e][1,3]oxa zines", INDIAN JOURNAL OF CHEMISTRY, SECTION B: ORGANIC CHEMISTRY INCLUDING MEDICINAL CHEMISTRY; CODEN: IJSBDB; ISSN: 0376-4699, Bd. 49B, Nr. 8, August 2010 (2010-08), Seiten 1083-1087, XP002667133,

## Beschreibung

Die vorliegende Erfindung betrifft neue Polytetrahydrobenzoxazine, welche durch das unten angegebene Herstellverfahren oder alternativ durch die unten angegebene allgemeine Strukturformel I definiert werden können. Die Polytetrahydrobenzoxazine können auch in quaternisierter Form vorliegen.

Weiterhin betrifft die vorliegende Erfindung neue Bistetrahydrobenzoxanzine, welche als Zwischenprodukte bei der Herstellung der Polytetrahydrobenzoxazine auftreten.

Weiterhin betrifft die vorliegende Erfindung die Verwendung der Polytetrahydrobenzoxazine und der Bistetrahydrobenzoxazine als Kraftstoffadditiv oder Schmierstoffadditiv, insbesondere als Detergensadditiv für Dieselkraftstoffe, vor allem für direkteinspritzende Dieselmotoren, sowie Additivkonzentrate, Kraftstoffzusammensetzungen und Schmierstoffzusammensetzungen, die die Polytetrahydrobenzoxazine bzw. Bistetrahydrobenzoxazine enthalten.

Bei direkteinspritzenden Dieselmotoren wird der Kraftstoff durch eine direkt in den Brennraum reichende Mehrloch-Einspritzdüse des Motors eingespritzt und feinst verteilt (vernebelt), anstatt wie beim klassischen (Kammer-)Dieselmotor in eine Vor- oder Wirbelkammer eingeführt zu werden. Der Vorteil der direkteinspritzenden Dieselmotoren liegt in ihrer für Dieselmotoren hohen Leistung und einem dennoch geringen Verbrauch. Außerdem erreichen diese Motoren ein sehr hohes Drehmoment schon bei niedrigen Drehzahlen.

Zur Zeit werden im Wesentlichen drei Verfahren eingesetzt, um den Kraftstoff direkt in den Brennraum des Dieselmotors einzuspritzen: die konventionelle Verteilereinspritzpumpe, das Pumpe-Düse-System (Unit-Injector-System bzw. Unit-Pump-System) und das Common-Rail-System.

Beim Common-Rail-System wird der Dieselkraftstoff von einer Pumpe mit Drücken bis zu 2000 bar in eine Hochdruckleitung, die "Common-Rail", gefördert. Ausgehend von der Common-Rail laufen Stichleitungen zu den verschiedenen Injektoren, die den Kraftstoff direkt in den Brennraum injizieren. Dabei liegt auf der Common-Rail stets der volle Druck an, was eine Mehrfacheinspritzung oder eine spezielle Einspritzform ermöglicht. Bei den anderen Injektionssystemen ist dagegen nur eine geringere Variation der Einspritzung möglich. Die Einspritzung beim Common-Rail wird im Wesentlichen in drei Gruppen unterteilt: (1.) Voreinspritzung, durch die im Wesentlichen eine weichere Verbrennung erreicht wird, so dass harte Verbrennungsgeräusche ("Nageln") vermindert werden und der Motorlauf ruhig erscheint; (2.) Haupteinspritzung, die insbesondere für einen guten Drehmomentverlauf verantwortlich ist; und (3.) Nacheinspritzung, die insbesondere für einen geringen Stickoxid-Wert im Abgas sorgt. Bei dieser Nacheinspritzung wird der Kraftstoff in der Regel nicht verbrannt, sondern durch Restwärme im Zylinder verdampft. Das dabei gebildete Abgas-/Kraftstoffgemisch wird zur Abgasanlage transportiert, wo der Kraftstoff in Gegenwart geeigneter Katalysatoren als Reduktionsmittel für die Stickoxide wirkt.

Durch die variable, zylinderindividuelle Einspritzung kann beim Common-Rail-Einspritzsystem der Schadstoffausstoß des Motors, z.B. der Ausstoß von Stickoxiden (NOₓ), Kohlenmonoxid (CO) und insbesondere von Partikeln (Ruß), positiv beeinflusst werden. Dies ermöglicht beispielsweise, dass mit Common-Rail-Einspritzsystemen ausgerüstete Motoren der Euro 4-Norm theoretisch auch ohne zusätzlichen Partikelfilter genügen können.

In modernen Common-Rail-Dieselmotoren können sich unter bestimmten Bedingungen, beispielsweise bei Verwendung von biodieselhaltigen Kraftstoffen oder von Kraftstoffen mit Metall-Verunreinigungen wie Zink-Verbindungen, Kupfer-Verbindungen, Blei-Verbindungen und weiteren Metallverbindungen, an den Injektoröffnungen Ablagerungen bilden, die das Einspritzverhalten des Kraftstoffs negativ beeinflussen und dadurch die Performance des Motors beeinträchtigen, d.h. insbesondere die Leistung verringern, aber zum Teil auch die Verbrennung verschlechtern. Die Bildung von Ablagerungen wird durch bauliche Weiterentwicklungen der Injektoren, insbesondere durch die Veränderung der Geometrie der Düsen (engere, konische Öffnungen mit abgerundetem Auslass) noch verstärkt. Für eine dauerhaft optimale Funktionsweise von Motor und Injektoren müssen solche Ablagerungen in den Düsenöffnungen durch geeignete Kraftstoffadditive verhindert oder reduziert werden.

In der WO 2009/040582 wird die Verwendung eines Mannich-Reaktionsproduktes aus einem Aldehyd, einem Polyamin und einem substituiertem Phenol mit einem Substituenten, der ein mittleres Molekulargewicht von weniger als 300 aufweist, als Dieseladditiv zur Verbesserung der Wirkung des Dieselmotors, insbesondere die Verringerung des Leistungsabfall im Motor und die Verringerung von Ablagerungen auf den Injektoren, beschrieben.

In der WO 2009/040583 wird die Verwendung der Kombination eines Mannich-Reaktionsproduktes aus einem Aldehyd, einem Polyamin und einem substituierten Phenol mit einem Polyisobutylsuccinimid als Dieseladditiv zur Verbesserung der Wirkung des Dieselmotors, insbesondere die Verringerung des Leistungsabfall im Motor und die Verringerung von Ablagerungen auf den Injektoren, beschrieben.

In der WO 2009/040584 wird ein metallhaltiger Dieselkraftstoff beschrieben, welcher als wirkungsverbesserndes Additiv ein Mannich-Reaktionsprodukt aus einem Aldehyd, einem Polyamin der Struktur eines gegebenenefalls substituierten Ethylendiamins und einem substituiertem Phenol enthält. Die Wirkungsverbesserung besteht speziell in der Verringerung des Leistungsabfall im Motor, der Verringerung von Ablagerungen auf den Injektoren, der Verringerung von Ablagerungen im Kraftstofffilter und der Verringerung des Kraftstoffverbrauches.

In der WO 2009/040585 wird die Verwendung eines Mannich-Reaktionsproduktes aus einem Aldehyd, einem Polyamin und einem substituiertem Phenol, wobei das Molverhältnis von Phenol zu Polyamin in der Reaktionsmischung wenigstens 1,5 : 1 beträgt, als Dieseladditiv zur Verbesserung der Wirkung des Dieselmotors, insbesondere die Verringerung des Leistungsabfalls im Motor, die Verringerung von Ablagerungen auf den Injektoren, die Verringerung von Ablagerungen im Kraftstofffilter und die Verringerung des Kraftstoffverbrauches, beschrieben.

Die GB-A 2 468 130 offenbart einen Dieselkraftstoff, der ein oder mehrere wirkungsverstärkende Additive aus der Gruppe der gegebenenfalls polymeren Phenol-Ammoniak-Aldehyd-Mannichaddukte, der Polyisobutylsuccinimide, der Antioxidantien, der Mischungen von Polyisobutylsuccinimiden mit Polyether-Trägerölen und der Phenol-Polyamin-Aldehyd-Mannichaddukte enthält. Diese Additive bewirken eine Verbesserung der Wirkung des Dieselmotors und eine Verringerung von Ablagerungen im Dieselmotor.

In der WO 2008/027881 werden quaternäre Ammoniumsalze als Reaktionsprodukte aus Mannichaddukten, welche eine tertiäre Aminogruppe aufweisen und aus einem substituierten Phenol, einem Aldehyd und einem Amin erhältlich sind, und einem Quaternisierungsmittel beschrieben. Diese quaternären Ammoniumsalze eignen sich unter anderem als Additive in Kraftstoffen.

Die im Stand der Technik beschriebenen Additivsysteme weisen jedoch eine Reihe von Nachteilen - insbesondere bei direkteinspritzenden Dieselmotoren, vor allem bei solchen mit Common-Rail-Einspritzsystemen - auf. Es treten noch zu starke Ablagerungen in den Einspritzsystemen der Motoren auf und der Kraftstoffverbrauch sowie der Leistungsverlust (powerloss) sind noch zu hoch. Weiterhin weisen die mit den im Stand der Technik beschriebenen Additivsystemen additivierten Dieselkraftstoffe noch verbesserungsbedürftige Tieftemperatureigenschaften auf. Auch ist die Verträglichkeit von Motorenölen mit den im Stand der Technik beschriebenen Additivsystemen noch nicht optimal.

Es besteht daher die Aufgabe, in ihrer Wirkung verbesserte Kraft- und Schmierstoffadditive bereitzustellen. Die Aufgabe wurde durch die nachfolgend beschriebenen Polytetrahydrobenzoxazine und Bistetrahydrobenzoxazine gelöst.

Die erfindungsgemäßen Polytetrahydrobenzoxazine können durch ihr Herstellverfahren definiert werden. Demgemäß sind Gegenstand der vorliegenden Erfindung Polytetrahydrobenzoxazine, welche durch die Reaktionsschritte
(A) Umsetzung mindestens eines Diamins der allgemeinen Formel H₂N-A-NH₂, in der das Brückenglied A für C₁- bis C₂₀-Alkylen, welches durch bis zu 10 Sauerstoffatome und/oder tertiäre Stickstoffatome unterbrochen sein kann, C₂- bis C₂₀-Alkenylen, C₅- bis C₂₀-Cycloalkylen, C₆- bis C₂₀-Arylen oder C₇- bis C₂₀-Aralkylen steht, mit mindestens einem C₁- bis C₁₂-Aldehyd und mindestens einem C₁- bis C₈-Alkanol bei einer Temperatur von 20 bis 80°C unter Abspaltung und Entfernung von Wasser, wobei sowohl der Aldehyd als auch der Alkohol in jeweils mehr als der doppelten molaren Menge gegen über dem Diamin eingesetzt werden;
(B) Umsetzung des Kondensationsproduktes aus Reaktionsschritt (A) mit mindestens einem Phenol, welches mindestens einen langkettigen Substituenten mit 6 bis 30 Kohlenstoffatomen trägt, im stöchiometrischen Verhältnis zum ursprünglich in Schritt (A) eingesetzten Diamin von 1,2 : 1 bis 3,5 : 1 bei einer Temperatur von 30 bis 120°C;
(C) Erhitzen des Umsetzungsproduktes aus Reaktionsschritt (B) auf eine Temperatur von 125 bis 280°C für mindestens 10 Minuten
erhältlich sind.

C₁- bis C₂₀-Alkylen für das Brückenglied A steht für lineare oder ein- oder mehrfach verzweigte gesättigte Kohlenwasserstoff-Brückenglieder mit 1 bis 20, insbesondere 1 bis 10, vor allem 1 bis 4 Kohlenstoffatomen, beispielsweise -CH₂-, -(CH₂)₂-, -CH(CH₃)-, -(CH₂)₃-, -CH₂-CH(CH₃)-, -(CH₂)₄-, -CH₂CH₂-CH(CH₃)-, -CH₂-CH(CH₃)-CH₂-, -(CH₂)₅-, -CH₂-C(CH₃)₂-CH₂-, -(CH₂)₆-, -CH(CH₃)-(CH₂)₂-CH(CH₃)-, -CH(CH₃)-(CH₂)₃-CH(CH₃)-, -(CH₂)₇- oder -(CH₂)₈-.

Im Falle einer Unterbrechung der C₁- bis C₂₀-Alkylen-Brückenglied durch bis zu 10, insbesondere durch bis zu 4, vor allem durch ein oder zwei oder drei Sauerstoffatome und/oder tertiäre Stickstoffatome kommen z. B. die folgenden Strukturen für A in Betracht: -CH₂-O-CH₂-, -CH₂CH₂-O-CH₂CH₂-, -CH₂-O-CH₂CH₂-O-CH₂-, -CH₂-O-CH₂CH₂-O-CH₂CH₂-O-CH₂-, -CH₂CH₂-O-CH₂CH₂-O-CH₂CH₂-, -CH₂CH₂-O-CH₂CH₂-O-CH₂CH₂-O-CH₂CH₂-, CH₂-N(CH₃)-CH₂- oder -CH₂CH₂-N(CH₃)-CH₂CH₂-. Die Seitenketten an unterbrechenden tertiären Stickstoffatomen werden üblicherweise von C₁- bis C₄-Alkylresten wie Methyl oder Ethyl gebildet; beim Auftreten solcher tertiären Stickstoffatome wird auch mit Einbezug der Alkyl-Seitenketten die maximale Kohlenstoffanzahl von 20, insbesondere von 10, vor allem von 4, nicht überschritten.

C₂- bis C₂₀-Alkenylen für das Brückenglied A steht für ein- oder mehrfach, insbesondere einfach ungesättigte Kohlenwasserstoff-Brückenglieder mit 1 bis 20, insbesondere 1 bis 10, vor allem 1 bis 4 Kohlenstoffatomen, beispielsweise -CH=CH-, -CH=CH-CH₂-, -CH=CH-CH₂-CH₂-, -CH₂-CH=CH-CH₂-, -CH=CH-CH=CH-, -CH(CH₃)-CH=CH- oder -CH₂-C(CH₃)=CH-.

C₅- bis C₂₀-Cycloalkylen, insbesondere C₅- bis C₈-Cycloalkylen, für das Brückenglied A steht beispielsweise für 1,1-, 1,2- oder 1,3-Cyclopentylen, 1,1-, 1,2-, 1,3- oder 1,4-Cyclohexylen, 1,1-, 1,2-, 1,3- oder 1,4-Cycloheptylen oder 1,1-, 1,2-, 1,3-, 1,4- oder 1,5-Cyclooctylen, welches zusätzlich ein oder mehrere C₁- bis C₄-Alkylsubstituenten wie Methyl- oder Ethylgruppen tragen kann, oder für ein Dicyclohexylmethan-Gerüst mit freien Valenzen in der 4- und 4'-Position der Cyclohexylringe.

C₆- bis C₂₀-Arylen, insbesondere C₆- bis C₁₄-Arylen, für das Brückenglied A steht beispielsweise für ortho-, meta- oder para-Phenylen, Naphtylene, Anthracylene, Phenanthrylene oder 4,4'-Diphenylen, die an ihren aromatischen Kernen zusätzlich ein oder mehrere C₁- bis C₄-Alkylsubstituenten wie Methyl- oder Ethylgruppen tragen können.

C₇- bis C₂₀-Aralkylen, insbesondere C₇- bis C₁₂-Aralkylen, für das Brückenglied A steht für Strukturen, deren eine freie Valenz von einem sp²-hybridisierten Kohlenstoffatom in einem aromatischen Ring und deren andere freie Valenz von einem sp³-hybridisierten Kohlenstoffatom in einer Seitenkette des aromatischen Ringes wie einem Phenylkern ausgeht oder deren beide freie Valenzen von sp³-hybridisierten Kohlenstoffatomen in verschiedenen Seitenketten eines aromatischen Ringes ausgeht, z. B. ortho-, meta- oder para-C₆H₄-CH₂-, ortho-, meta- oder para-C₆H₄-CH₂CH₂-, ortho-, meta- oder para-C₆H₄-(CH₂)₃-, ortho-, meta- oder para-C6H4-(CH2)4- oder ortho-, meta- oder para-CH2-C₆H₄-CH₂-.

In einer bevorzugten Ausführungsform sind die erfindungsgemäßen Polytetrahydrobenzoxazine in Reaktionsschritt (A) aus mindestens einem Diamin der allgemeinen Formel H₂N-(CH₂)ₓ-NH₂ [d. h. A = -(CH₂)ₓ-], in der x für eine Zahl von 1 bis 10 steht, erhältlich. Besonders bevorzugt sind Diamine der allgemeinen Formel H₂N-(CH₂)ₓ-NH₂, in der x für eine Zahl von 1 bis 8, insbesondere für eine Zahl von 1 bis 4, vor allem für die Zahl 2, steht. Für x = 2 ist das besagte Diamin 1,2-Ethylendiamin.

Als C₁- bis C₁₂-Aldehyde, insbesondere C₁- bis C₇-Aldehyde, eignen sich beispielsweise Formaldehyd, Acetaldehyd, Propionaldehyd, Butyraldehyd, Valeraldehyd oder Benzaldehyd. In einer bevorzugten Ausführungsform sind die erfindungsgemäßen Polytetrahydrobenzoxazine in Reaktionsschritt (A) aus Formaldehyd oder einer polymeren Form von Formaldehyd wie Paraformaldehyd oder 1,3,5-Trioxan erhältlich.

Als C₁- bis C₈-Alkanole, insbesondere C₁- bis C₄-Alkanol, eignen sich beispielsweise Methanol, Ethanol, n-Propanol, Isopropanol, n-Butanol, sec.-Butanol, Isobutanol, tert.-Butanol, n-Pentanol, sec.-Pentanol, Isopentanol, tert.-Pentanol, n-Hexanol, n-Heptanol, n-Octanol, 2-Ethylhexanol, n-Nonanol, Isononanol oder n-Decanol sowie auch Gemische aus solchen Alkanolen. In einer bevorzugten Ausführungsform sind die erfindungsgemäßen Polytetrahydrobenzoxazine in Reaktionsschritt (A) aus mindestens einem C₃- oder C₄-Alkanol erhältlich.

Die Umsetzung des Diamins der Formel H₂N-A-NH₂ mit dem C₁- bis C₁₂-Aldehyd und dem C₁- bis C₈-Alkanol erfolgt in der Regel bei Raumtemperatur bis leicht erhöhter Temperatur, d.h. bei 20 bis 80°C, insbesondere bei 25 bis 70°C, vor allem bei 30 bis 60°C. Vorzugsweise arbeitet man im schwachen Vakuum, d.h. bei 20 mbar bis Normaldruck, insbesondere bei 30 bis 700 mbar, vor allem bei 40 bis 500 mbar, um das abgespaltene Wasser besser aus dem Reaktionsgemisch - beispielsweise durch azeotrope Destilllation - entfernen zu können. Die optimalen Temperatur- und Druckeinstellungen hängen natürlich vom Siedepunkt des oder der eingesetzten C₁- bis C₈-Alkanole ab. Die oben angegebenen Vorzugsbereiche für Temperatur- und Druckeinstellung empfehlen sich in besonderem Maße bei Einsatz von C₃- oder C₄-Alkanolen.

Die Umsetzung des Diamins der Formel H₂N-A-NH₂ mit dem C₁- bis C₁₂-Aldehyd und dem C₁- bis C₈-Alkanol erfolgt vorteilhafterweise in einem inerten organischen Lösungsmittel oder einem Gemisch solcher Lösungsmittel, insbesondere einem Kohlenwasserstoff wie Hexan, Cyclohexan, Toluol oder Xylol oder einem Halogenkohlenwasserstoff wie Chloroform oder Chlorbenzol. In vielen Fällen hat es sich als günstig erwiesen, zuerst den C₁- bis C₁₂-Aldehyd und das C₁- bis C₈-Alkanol bei Raumtemperatur oder möglichst niedriger Temperatur im inerten Lösungsmittel vorzulegen, danach das Diamin zuzugeben und dann - gegebenenfalls im Vakuum - auf Umsetzungstemperatur zu erwärmen und das abgespaltene Wasser zu entfernen. Die Umsetzungsdauer beträgt typischerweise 1 bis 10 Stunden.

In einer bevorzugten Ausführungsform beträgt im Reaktionsschritt (A) das stöchiometrische Verhältnis von Diamin zu Aldehyd 1 : 4, wobei eine Abweichung von diesem Verhältnis von bis zu 10 % toleriert werden kann, und das Alkanol wird in der mindestens 3,5-fachen molaren, insbesondere 4-fach molaren Mengen gegenüber dem Diamin eingesetzt. Das Alkanol kann auch in einer höheren Menge, d. h. im Überschuss, eingesetzt werden, beispielsweise in der 4- bis 8-fachen molaren Menge gegenüber dem Diamin. Das bevorzugte stöchiometrische Verhältnis von Diamin zu Aldehyd liegt somit typischerweise im Bereich von 1 : (3,6 - 4,4) oder von (0,9 - 1,1) : 4, insbesondere von 1 : (3,9 - 4,1) oder von (0,97-1,03) : 4.

Im Reaktionsschritt (A) wird das aktive Agens für die Umsetzung mit dem Phenol im Reaktionsschritt (B) erzeugt, welches in der Regel eine Mischung aus dem teilweise oder vollständig hydroxyalkylierten Diamin, das teilweise oder vollständig mit dem C₁-bis C₈-Alkanol verethert vorliegt, und gegebenenfalls ringgeschlossenen Folgeprodukten wie Imidazolodinen darstellt. Die einzelnen Komponenten dieser Mischung stehen üblicherweise miteinander im chemischen Gleichgewicht, so dass normalerweise alle oder fast alle Komponenten dieser Mischung für die Weiterreaktion im nächsten Reaktionsschritt (B) im Sinne der vorliegenden Erfindung zur Verfügung stehen.

Eine solche Mischung, die zum Beispiel durch Umsetzung von Ethylendiamin, Formaldehyd und Isobutanol erhältlich ist, ist beispielhaft mit ihren Komponenten nachfolgend dargestellt:

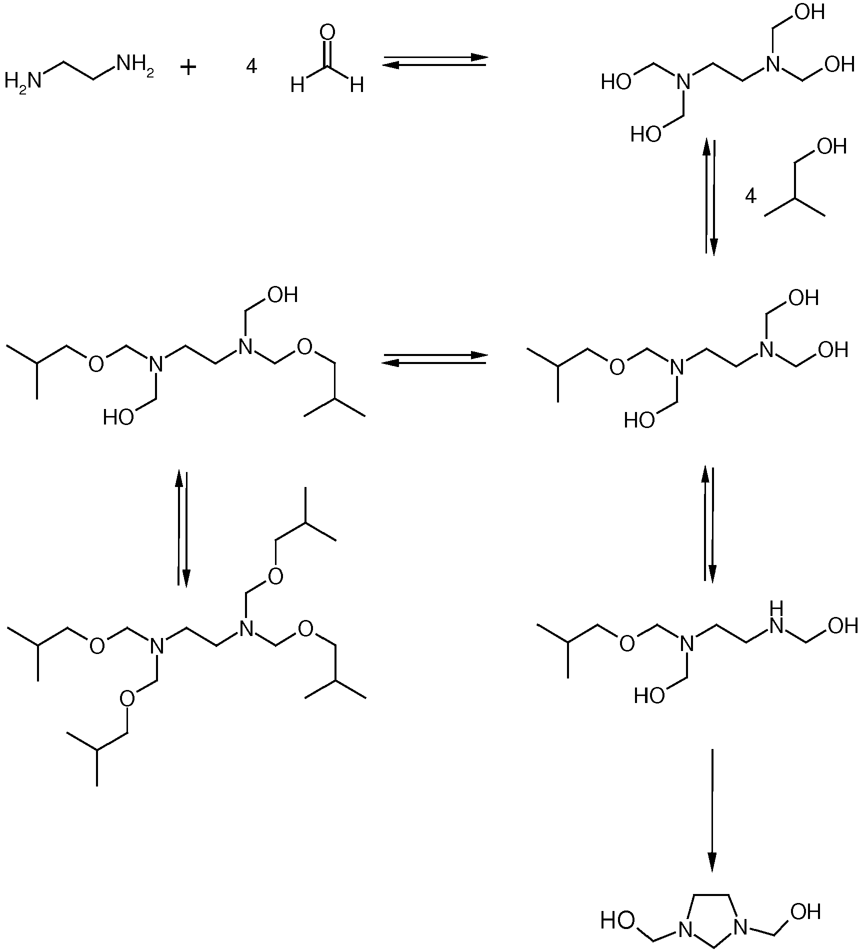

Das im Reaktionsschritt (B) eingesetzte Phenol trägt als den mindestens einen langkettigen Substituenten mit 6 bis 3000 Kohlenstoffatomen typischerweise einen entsprechenden Hydrocarbylrest. Unter einem Hydrocarbylrest soll hier ein Kohlenwasserstoffrest jeglicher Struktur verstanden werden, der jedoch in untergeordneter Menge noch Heteroatome wie Sauerstoff- und/oder Stickstoffatome und/oder Halogenatome enthalten und/oder funktionelle Gruppen wie Hydroxylgruppen, Carboxylgruppen, Carbonsäureestergruppen, Cyanogruppen, Nitrogruppen und/oder Sulfogruppen tragen kann. Der besagte langkettige Hydrocarbylrest kann gesättigter oder ungesättigter Natur sein; er kann linear oder verzweigt aufgebaut sein; er kann aromatische und/oder heterocyclische Substrukturen enthalten. Der mindestens eine langkettige Substituent am Phenol ist dient hauptsächlich dazu, die erfindungsgemäßen Polytetrahydrobenzoxazine in Mineralölprodukten wie Kraftstoffen und Schmierstoffen besser löslich zu machen.

Dieser längerkettige Hydrocarbylrest am Phenol ist vorzugsweise ein Hydrocarbylrest mit 6 bis 30 Kohlenstoffatomen oder ein Polyisobutylrest mit 16 bis 3000 Kohlenstoffatomen.

Als Hydrocarbylreste mit 6 bis 30 Kohlenstoffatomen am Phenol kommen vorzugsweise C₆- bis C₃₀-Alkenylreste, insbesondere C₇- bis C₂₀-Alkenylreste, vor allem C₈- bis C₁₈-Alkenylreste, und in ganz besonderem Maße C₆- bis C₃₀-Alkylreste, insbesondere C₇- bis C₁₈-Alkylreste, vor allem C₈- bis C₁₂-Alkylreste, in Betracht. Das Phenol kann einen, zwei oder drei solcher langkettigen Substituenten tragen, bevorzugt trägt das Phenol einen solchen langkettigen Substituenten. Neben den langkettigen Substituenten kann das Phenol auch noch einen, zwei oder drei kürzerkettige Alkyl- oder Alkenylreste wie Methyl-, Ethyl-, n-Propyl-, Isopropyl-, n-Butyl-, sec.-Butyl-, Isobutyl-, tert.-Butyl-, Vinyl- oder Allylreste und/oder eine, zwei oder drei funktionelle Gruppen wie Halogenatome, z. B. Chlor oder Brom, Nitrogruppen, Cyanogruppen, Carboxylgruppen, Carbonsäureestergruppen oder Sulfogruppen tragen, wobei die Gesamtzahl der Substituenten am Phenol nicht mehr als 5, vorzugsweise nicht mehr als 4, vor allem nicht mehr als 3 beträgt.

Beispiele für die genannten Phenole mit mindestens einem langkettigen Substituenten mit 6 bis 30 Kohlenstoffatomen sind Phenole mit einem n-Hexyl-, n-Heptyl-, n-Octyl-, tert.-Octyl-, 2-Ethylhexyl-, n-Nonyl-, Isononyl-, n-Decyl-, 2-Propylheptyl-, n-Undecyl-, n-Dodecyl-, n-Tridecyl-, Isotridecyl-, n-Tetradecyl-, n-Hexadecyl-, n-Octadecyl-, Oleyl-, Linolyl- oder Linolenylrest in ortho- oder para-Stellung, weiterhin ortho-Kresol mit einem der oben genannten langkettigen Alkyl- oder Alkenylreste in 4- oder 6-Stellung, meta-Kresol mit einem der oben genannten langkettigen Alkyl- oder Alkenylreste in 4- oder 6-Stellung, para-Kresol mit einem der oben genannten langkettigen Alkyl- oder Alkenylreste in 2- oder 6-Stellung sowie Phenole mit zwei gleichen oder verschiedenen oben genannten langkettigen Alkyl- oder Alkenylreste in 2- und 4-Stellung.

Im Falle von Polyisobutylresten umfasst diese vorzugsweise 21 bis 1000, insbesondere 26 bis 3000 oder insbesondere 26 bis 500, vor allem 30 bis 3000 oder vor allem 30 bis 250 Kohlenstoffatome oder sie weisen zahlenmittlere Molekulargewichte Mₙ von 183 bis 42.000, vorzugsweise 500 bis 15.000, insbesondere 700 bis 7000, vor allem 900 bis 3000, ganz besonders bevorzugt 900 bis 1100 auf.

Das Phenol kann einen, zwei oder drei solcher Polyisobutylreste tragen, bevorzugt trägt das Phenol einen solchen Polyisobutylrest. Neben den Polyisobutylresten kann das Phenol auch noch einen, zwei oder drei kürzerkettige Hydrocarbylreste wie Methyl-, Ethyl-, n-Propyl-, Isopropyl-, n-Butyl-, sec.-Butyl-, Isobutyl-, tert.-Butyl-, Vinyl-, Allyl-, n-Pentyl, sec.-Pentyl-, Isopentyl-, tert.-Pentyl-, n-Hexyl-, n-Heptyl-, n-Octyl-, tert.-Octyl-, 2-Ethylhexyl-, n-Nonyl-, Isononyl-, n-Decyl-, 2-Propylheptyl-, n-Undecyl-, n-Dodecyl-, n-Tridecyl-, Isotridecyl-, n-Tetradecyl-, n-Hexadecyl-, n-Octadecyl-, Oleyl-, Linolyl- oder Linolenylreste und/oder eine, zwei oder drei funktionelle Gruppen wie Halogenatome, z. B. Chlor oder Brom, Nitrogruppen, Cyanogruppen, Carboxylgruppen, Carbonsäureestergruppen oder Sulfogruppen tragen, wobei die Gesamtzahl der Substituenten am Phenol nicht mehr als 5, vorzugsweise nicht mehr als 4, vor allem nicht mehr als 3 beträgt.

In einer bevorzugten Ausführungsform sind die erfindungsgemäßen Polytetrahydrobenzoxazine in Reaktionsschritt (B) aus mindestens einem Phenol, welches in para-Stellung (4-Stellung) zur Hydroxylgruppe einen C₈- bis C₁₂-Alkylrest oder eine Polyisobutylrest mit 16 bis 3000 Kohlenstoffatomen trägt, erhältlich.

Die Umsetzung des Kondensationsproduktes aus Reaktionsschritt (A) mit dem mindestens einen langkettig-substituierten Phenol erfolgt gemäß Reaktionsschritt (B) bei höheren Temperaturen als in Reaktionsschritt (A), d.h. bei 30 bis 120°C, insbesondere bei 35 bis 105°C, vor allem bei 40 bis 90°C. Vorzugsweise arbeitet man bei Normaldruck. Die Umsetzung erfolgt vorteilhafterweise in einem inerten organischen Lösungsmittel oder einem Gemisch solcher Lösungsmittel, insbesondere einem aromatischen Kohlenwasserstoff wie Toluol, Xylol oder einem technischen Gemisch höhersiedender aromatische Kohlenwasserstoffe, z. B. Solvesso™ 100, 150, 200, 150 ND oder 200 ND. Die Umsetzungsdauer beträgt typischerweise 1 bis 10 Stunden. Das stöchiometrische Verhältnis von Phenol zu in Reaktionsschritt (A) eingesetztem Diamin beträgt vorzugsweise 1,5 :1 bis 3,0 : 1, insbesondere 1,75 :1 bis 2,75 : 1, vor allem 1,9 : 1 bis 2,6 : 1.

Das in Reaktionsschritt (B) erzeugte Produkt hat oder hat überwiegend die Struktur eines Bistetrahydrobenzoxazins der allgemeinen Formel II 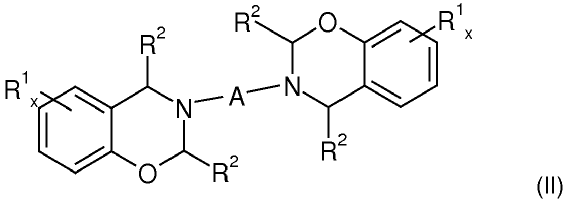 in der
x für die Zahl 1, 2 oder 3 steht,
R¹ gleiche oder verschiedene C₁- bis C₃₀₀₀-Hydrocarbylreste bezeichnet, wobei jeder Benzolkern mindestens einen Hydrocarbylrest trägt,
R² Wasserstoff oder gleiche oder verschiedene C₁- bis C₁₁-Alkylreste bezeichnet und
A ein Brückenglied mit 1 bis 20 Kohlenstoffatomen bedeutet,
wobei R¹ die Substituenten des eingesetzten Phenols sind, R² den Rest des eingesetzten Aldehyds darstellt und A dem Brückenglied A in der allgemeinen Formel für das Diamin H₂N-A-NH₂ entspricht.

In den Verbindungen II können auch verschiedene Substituenten R¹ auftreten, wenn Gemische unterschiedlicher Phenole im Reaktionsschritt (B) eingesetzt werden.

Ein typisches Beispiel für ein Bistetrahydrobenzoxazins der allgemeinen Formel II ist die nachfolgend wiedergegebene Verbindung der Formel IIa: 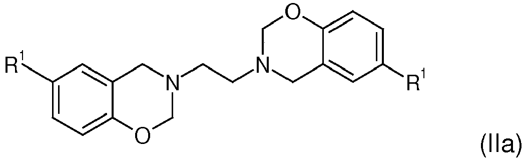 in der R¹ z. B. für tert.-Octyl, n-Nonyl, n-Dodecyl oder Polyisobutyl mit einem Mₙ von 1000 steht.

Der Reaktionsschritt (C) wird durch Erhitzen des Umsetzungsproduktes aus Reaktionsschritt (B) auf Temperaturen deutlich über denen des Schrittes (B) vorgenommen. Hierbei arbeitet man vorzugsweise bei 150 bis 250°C, insbesondere bei 175 bis 230°C, vor allem bei 190 bis 220°C, und vorzugsweise bei Normaldruck. Das Erhitzen auf den angegebenen Temperaturbereich erfolgt für mindestens 10 Minuten, vorzugsweise für mindestens 30 Minuten, vor allem für 45 bis 120 Minuten. Im wesentlichen polymerisieren die Bistetrahydrobenzoxazine II dabei unter Öffnung von Tetrahydrooxazin-Ringen und bilden ein stark verzweigtes -jedoch nicht bis zur Schwer- oder Unlöslichkeit in Mineralölmedien vernetztes - zwei- bis dreidimensionales Polymersystem.

Das Erhitzen des Umsetzungsproduktes aus Reaktionsschritt (B) gemäß Reaktionsschritt (C) erfolgt vorteilhafterweise in einem inerten organischen Lösungsmittel oder einem Gemisch solcher Lösungsmittel, insbesondere einem aromatischen Kohlenwasserstoff wie Toluol, Xylol oder einem technischen Gemisch höhersiedender aromatische Kohlenwasserstoffe, z. B. Solvesso™ 100, 150, 200, 150 ND oder 200 ND.

Ein typisches Beispiel für ein in Reaktionsschritt (C) gebildetes Polytetrahydrobenzoxazin ist nachfolgend als Verbindung der allgemeinen Formel la wiedergegeben:

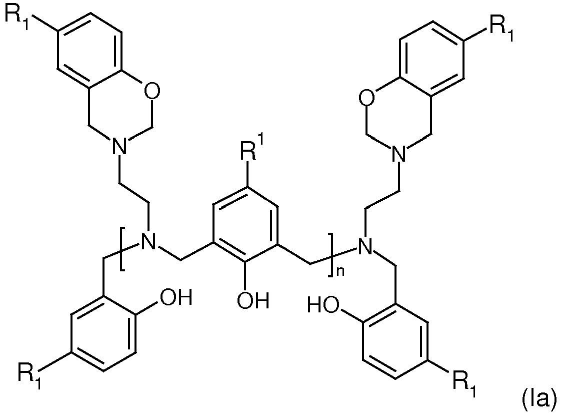

Die Substituenten R¹ haben die oben angegebene Bedeutung; es können auch verschiedene Substituenten R¹ im Molekül auftreten, wenn Gemische unterschiedlicher Phenole im Reaktionsschritt (B) eingesetzt werden. Die Laufzahl n nimmt typischerweise Werte von 2 bis 10, insbesondere 4 bis 8, an.

In den erfindungsgemäßen Polytetrahydrobenzoxazinen bestehen die Enden der Seitenketten meist noch aus geschlossenen Tetrahydrooxazin-Ringen.

Die erfindungsgemäßen Polytetrahydrobenzoxazine weisen vorzugsweise ein zahlenmittleres Molekulargewicht (Mₙ) von 700 bis 50.000, insbesondere von 1500 bis 25.000, vor allem von 2500 bis 10.000, und einen Polydispersitätsindex (PDI) von 1,5 bis 7,5, vorzugsweise von 2,0 bis 5,0, auf.

Zur Modifikation oder Verbesserung der Wirksamkeit als Kraftstoff- oder Schmierstoffadditive können die beschriebenen Polytetrahydrobenzoxazine nachträglich quaternisiert werden. Daher sind auch Gegenstand der vorliegenden Erfindung quaternisierte Polytetrahydrobenzoxazine, welche durch die beschriebenen Reaktionsschritte (A), (B) und (C) und zusätzlich den Reaktionsschritt
(D) Quaternisierung eines Teiles oder aller quaternisierbaren Aminofunktionen des Umsetzungsproduktes aus Reaktionsschritt (C)
erhältlich sind. Die quaternisierbaren Aminofunktionen in den beschriebenen Polytetrahydrobenzoxazinen sind die tertiären Stickstoffatome.

Als Quaternisierungsmittel kommen im Prinzip alle als solche geeigneten Verbindungen in Betracht. In einer bevorzugten Ausführungsform sind die erfindungsgemäßen quaternisierten Polytetrahydrobenzoxazine im Reaktionsschritt (D) durch Quaternisierung mit mindestens einem Epoxid erhältlich.

Vorzugsweise ist dieses Epoxid ein Hydrocarbyl-Epoxid, dessen vier Substituenten gleich oder verschieden sind und für Wasserstoff oder für Hydrocarbylreste stehen, wobei die Hydrocarbylreste wenigstens 1 bis 10 Kohlenstoffatome aufweist und mindestens ein solcher Hydrocarbylrest vorhanden sein muss. Insbesondere sind dies aliphatische oder aromatische Reste wie z.B. lineare oder verzweigte C₁- bis C₁₀-Alkylreste oder aromatische Reste wie Phenyl oder C₁- bis C₄-Alkylphenyl.

Als solche Hydrocarbyl-Epoxide eignen sich beispielsweise aliphatische und aromatische Alkylenoxide, wie insbesondere C₂- bis C₁₂-Alkylenoxide, z. B. Ethylenoxid, Propylenoxid, 1,2-Butylenoxid, 2,3-Butylenoxid, 2-Methyl-1,2-propenoxid (Isobutenoxid), 1,2-Pentenoxid, 2,3-Pentenoxid, 2-Methyl-1,2-butenoxid, 3-Methyl-1,2-butenoxid, 1,2-Hexenoxid, 2,3-Hexenoxid, 3,4-Hexenoxid, 2-Methyl-1,2-pentenoxid, 2-Ethyl-1,2-butenoxid, 3-Methyl-1,2-pentenoxid, 1,2-Decenoxid, 1,2-Dodecenoxid oder 4-Methyl-1,2-pentenoxid, sowie aromatensubstituierte Ethylenoxide wie gegebenenfalls substituiertes Styroloxid, insbesondere Styroloxid oder 4-Methylstyroloxid.

Im Falle der Verwendung von Epoxiden als Quaternisierungsmittel werden diese meist in Anwesenheit von freien Säuren, insbesondere in Anwesenheit von freien Protonensäuren wie vor allem mit C₁- bis C₁₂-Monocarbonsäuren, z. B. Ameisensäure, Essigsäure oder Propionsäure, oder C₂- bis C₁₂-Dicarbonsäuren, z. B. Oxalsäure oder Adipinsäure, oder auch in Abwesenheit von Sulfonsäuren, z. B. Benzolsulfonsäure oder Toluolsulfonsäure, oder wässrigen Mineralsäuren, z. B. Schwefelsäure oder Salzsäure, eingesetzt.

Zur Durchführung der Quaternisierung versetzt man das Polytetrahydrobenzoxazin aus Reaktionsschritt (C) üblicherweise mit wenigstens einem Epoxid, insbesondere in den erforderlichen stöchiometrischen Mengen, um die gewünschte Quaternisierung zu erreichen. Pro Äquivalent an quaternisierbarem tertiären Stickstoffatom kann man z. B. 0,1 bis 1,5 Äquivalente, oder 0,5 bis 1,25 Äquivalente, an Quaternisierungsmittel einsetzen. Insbesondere werden aber etwa annähernd äquimolare Anteile des Epoxids eingesetzt, um eine tertiäre Amingruppe zu quaternisieren. Man arbeitet hierbei typischerweise bei Temperaturen im Bereich von 15 bis 90°C, insbesondere von 20 bis 80°C oder von 30 bis 70°C. Die Reaktionsdauer kann dabei im Bereich von wenigen Minuten oder einigen Stunden, wie beispielsweise etwa 10 Minuten bis zu etwa 24 Stunden, liegen. Der Umsetzung kann dabei bei etwa 0,1 bis 20 bar, wie beispielsweise 1 bis 10 oder 1,5 bis 3 bar Druck, insbesondere aber etwa bei Normaldruck erfolgen. Insbesondere ist eine Inertgas-Atmosphäre, z. B. Stickstoff, zweckmäßig.

Falls erforderlich können die Reaktanden in einem geeigneten inerten organischen aliphatischen oder aromatischen Lösungsmittel oder einem Gemisch solcher Lösungsmittel für die Quaternisierung vorgelegt werden, oder es ist noch eine ausreichender Anteil an Lösungsmittel aus Reaktionsschritt (C) vorhanden. Typische Beispiele für geeignete Lösungsmittel sind diejenigen der oben genannten Solvesso™-Serie sowie Toluol oder Xylol.

Gegenstand der vorliegenden Erfindung ist auch ein Verfahren zur Herstellung von Polytetrahydrobenzoxazinen, welches dadurch gekennzeichnet, dass man sukzessive die bereits oben ausführlich beschriebenen Reaktionsschritte, nämlich
(A) Umsetzung mindestens eines Diamins der allgemeinen Formel H₂N-A-NH₂, in der das Brückenglied A für C₁- bis C₂₀-Alkylen, welches durch bis zu 10 Sauerstoffatome und/oder tertiäre Stickstoffatome unterbrochen sein kann, C₂- bis C₂₀-Alkenylen, C₅- bis C₂₀-Cycloalkylen, C₆- bis C₂₀-Arylen oder C₇- bis C₂₀-Aralkylen steht, mit mindestens einem C₁- bis C₁₂-Aldehyd und mindestens einem C₁- bis C₈-Alkanol bei einer Temperatur von 20 bis 80°C unter Abspaltung und Entfernung von Wasser, wobei sowohl der Aldehyd als auch der Alkohol jeweils in mehr als der doppelten molaren Menge gegen über dem Diamin eingesetzt werden;
(B) Umsetzung des Kondensationsproduktes aus Reaktionsschritt (A) mit mindestens einem Phenol, welches mindestens einen langkettigen Substituenten mit 6 bis 3000 Kohlenstoffatomen trägt, im stöchiometrischen Verhältnis zum ursprünglich in Schritt (A) eingesetzten Diamin von 1,2 : 1 bis 3,5 : 1 bei einer Temperatur von 30 bis 120°C;
(C) Erhitzen des Umsetzungsproduktes aus Reaktionsschritt (B) auf eine Temperatur von 125 bis 280°C für mindestens 10 Minuten
durchführt.

In einer bevorzugten Ausführungsform ist dieses Verfahren zur Herstellung von quaternisierten Polytetrahydrobenzoxazinen weiterhin dadurch gekennzeichnet, dass man sukzessive die Reaktionsschritte (A), (B) und (C) und zusätzlich den bereits oben auch ausführlich beschriebenen Reaktionsschritt, nämlich
(D) Quaternisierung eines Teiles oder aller quaternisierbaren Aminofunktionen des Umsetzungsproduktes aus Reaktionsschritt (C)
durchführt.

Die erfindungsgemäßen Polytetrahydrobenzoxazine können alternativ auch durch ihre allgemeine chemische Struktur definiert werden. Demgemäß sind Gegenstand der vorliegenden Erfindung Polytetrahydrobenzoxazine der allgemeinen Formel I 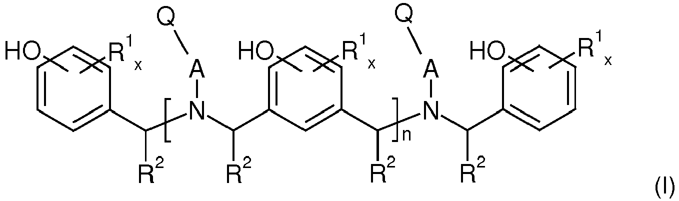 in der
x für die Zahl 1, 2, 3 oder 4 steht, wobei die Werte für x an den verschiedenen aromatischen Ringen unterschiedlich sein können,
n für eine ganze Zahl von 2 bis 10, insbesondere von 4 bis 8, steht,
R¹ gleiche oder verschiedene C₁- bis C₃₀₀₀-Hydrocarbylreste bezeichnet, wobei jeder Benzolkern mindestens einen Hydrocarbylrest trägt,
R² Wasserstoff oder gleiche oder verschiedene C₁- bis C₁₁-Alkylreste bezeichnet,
A ein Brückenglied mit 2 bis 20 Kohlenstoffatomen bedeutet und
Q den über ein Stickstoffatom angebundenen Rest einer Tetrahydrobenzoxazin-Einheit bezeichnet, welche in cyclischer Form gemäß der Formel 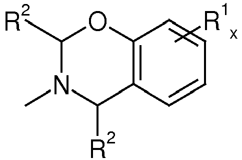 , wobei die Variablen R¹, R² und x die oben genannten Bedeutungen haben. Dabei sind R¹ die Substituenten des im oben beschriebenen Reaktionsschritt (B) eingesetzten Phenols, R² stellt den Rest des im oben beschriebenen Reaktionsschritt (A) eingesetzten Aldehyds dar und A entspricht dem Brückenglied A in der allgemeinen Formel für das im oben beschriebenen Reaktionsschritt (A) eingesetzte Diamin H₂N-A-NH₂.

Da die beschriebenen Bistetrahydrobenzoxazine als Zwischenprodukte und auch als potentielle Kraftstoff- und Schmierstoffadditive neue Verbindungen darstellen, sind ebenfalls Gegenstand der vorliegenden Erfindung Bistetrahydrobenzoxazine der allgemeinen Formel II 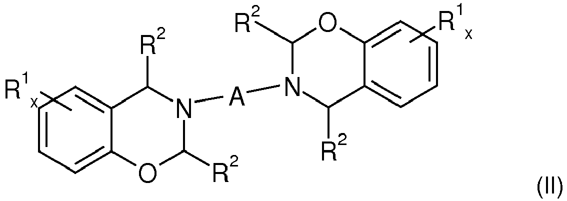 in der
x für die Zahl 1, 2, 3 oder 4 steht, wobei die Werte für x an den beiden aromatischen Ringen unterschiedlich sein können,
R¹ gleiche oder verschiedene C₁- bis C₃₀₀₀-Hydrocarbylreste bezeichnet, wobei jeder Benzolkern mindestens einen Hydrocarbylrest trägt, ausgewählt aus der Gruppe bestehend aus Polyisobutylrest mit 16 bis 3000 Kohlenstoffatomen, n-Nonyl-, Isononyl-, n-Decyl-, 2-Propylheptyl-, n-Undecyl-, n-Dodecyl-, n-Tridecyl-, Isotridecyl-, n-Tetradecyl-, n-Hexadecyl-, n-Octadecyl-, Oleyl-, Linolyl- oder Linolenylreste,
R² Wasserstoff oder gleiche oder verschiedene C₁- bis C₁₁-Alkylreste bezeichnet und
A ein Brückenglied mit 2 bis 20 Kohlenstoffatomen bedeutet,
wobei R¹ die Substituenten des im oben beschriebenen Reaktionsschritt (B) eingesetzten Phenols sind, R² den Rest des im oben beschriebenen Reaktionsschritt (A) eingesetzten Aldehyds darstellt und A dem Brückenglied A in der allgemeinen Formel für das im oben beschriebenen Reaktionsschritt (A) eingesetzte Diamin H₂N-A-NH₂ entspricht.

Die erfindungsgemäßen Polytetrahydrobenzoxazine und quaternisierten Polytetrahydrobenzoxazine und die erfindungsgemäßen Bistetrahydrobenzoxazine eignen sich in hervorragender Weise als Kraftstoffadditiv oder Schmierstoffadditiv. Kraftstoffe, in denen die erfindungsgemäßen Polytetrahydrobenzoxazine oder quaternisierten Polytetrahydrobenzoxazine oder die erfindungsgemäßen Bistetrahydrobenzoxazine als Kraftstoffadditiv eingesetzt werden können, sind hier insbesondere Ottokraftstoffe und Mitteldestillat-Kraftstoffe, hier vor allem Dieselkraftstoffe und Heizöle.

In ganz besonderem Maße eignen sich die erfindungsgemäßen Polytetrahydrobenzoxazine und quaternisierten Polytetrahydrobenzoxazine und die erfindungsgemäßen Bistetrahydrobenzoxazine als Detergensadditiv für Dieselkraftstoffe.

Speziell in ihrer Eigenschaft als Detergensadditiv für Dieselkraftstoffe finden die erfindungsgemäßen Polytetrahydrobenzoxazine und quaternisierten Polytetrahydrobenzoxazine und die erfindungsgemäßen Bistetrahydrobenzoxazine Verwendung als Additiv zur Verringerung oder Vermeidung von Ablagerungen in Einspritzsystemen von direkteinspritzenden Dieselmotoren, insbesondere in Common-Rail-Einspritzsystemen, zur Verringerung des Kraftstoffverbrauches von direkteinspritzenden Dieselmotoren, insbesondere von Dieselmotoren mit Common-Rail-Einspritzsystemen, und/oder zur Minimierung des Leistungsverlustes (powerloss) in direkteinspritzenden Dieselmotoren, insbesondere in Dieselmotoren mit Common-Rail-Einspritzsystemen.

Gegenstand der vorliegenden Erfindung ist auch ein Additivkonzentrat, das in Kombination mit weiteren Kraftstoffadditiven, insbesondere Dieselkraftstoffadditiven, wenigstens ein erfindungsgemäßes Polytetrahydrobenzoxazin oder quaternisiertes Polytetrahydrobenzoxazin oder ein erfindungsgemäßes Bistetrahydrobenzoxazin enthält.

Die erfindungsgemäßen Polytetrahydrobenzoxazine oder quaternisierten Polytetrahydrobenzoxazin oder die erfindungsgemäßen Bistetrahydrobenzoxazine liegen im erfindungsgemäßen Additivkonzentrat vorzugsweise in einer Menge von 0,1 bis 100 Gew.-%, besonders bevorzugt von 1 bis 80 Gew.-% und insbesondere von 10 bis 70 Gew.-%, bezogen auf das Gesamtgewicht des Konzentrats, vor.

Gegenstand der vorliegenden Erfindung ist weiterhin eine Kraftstoffzusammensetzung, insbesondere eine Dieselkraftstoffzusammensetzung, die in einer Hauptmenge eines üblichen Grundkraftstoffes, insbesondere eines Dieselkraftstoffes, eine wirksame Menge wenigstens eines erfindungsgemäßen Polytetrahydrobenzoxazins oder quaternisierten Polytetrahydrobenzoxazins oder eines erfindungsgemäßen Bistetrahydrobenzoxazins enthält.

Gegenstand der vorliegenden Erfindung ist weiterhin eine Schmierstoffzusammensetzung, die in einer Hauptmenge einer üblichen Schmierstoffformulierung eine wirksame Menge wenigstens eines erfindungsgemäßen Polytetrahydrobenzoxazins oder quaternisierten Polytetrahydrobenzoxazins oder eines erfindungsgemäßen Bistetrahydrobenzoxazins enthält.

Als Ottokraftstoffe kommen alle handelsüblichen Ottokraftstoffzusammensetzungen in Betracht. Als typischer Vertreter soll hier der marktübliche Eurosuper Grundkraftstoff gemäß EN 228 genannt werden. Weiterhin sind auch Ottokraftstoffzusammensetzungen der Spezifikation gemäß WO 00/47698 mögliche Einsatzgebiete für die vorliegende Erfindung.

Als Mitteldestillat-Kraftstoffe kommen alle handelsüblichen Dieselkraftstoff- und Heizölzusammensetzungen in Betracht. Bei Dieselkraftstoffen handelt es sich üblicherweise um Erdölraffinate, die in der Regel einen Siedebereich von 100 bis 400°C haben. Dies sind meist Destillate mit einem 95%-Punkt bis zu 360°C oder auch darüber hinaus. Dies können aber auch sogenannte "Ultra low sulfur diesel" oder "City diesel" sein, gekennzeichnet durch einen 95%-Punkt von beispielsweise maximal 345°C und einem Schwefelgehalt von maximal 0,005 Gew.-% oder durch einen 95%-Punkt von beispielsweise 285°C und einem Schwefelgehalt von maximal 0,001 Gew.-%. Neben den durch Raffination erhältlichen Dieselkraftstoffen, deren Hauptbestandteile längerkettige Paraffine darstellen, sind solche, die durch Kohlevergasung oder Gasverflüssigung ["gas to liquid" (GTL) Kraftstoffe] erhältlich sind, geeignet. Geeignet sind auch Mischungen der vorstehend genannten Dieselkraftstoffe mit regenerativen Kraftstoffen wie Biodiesel oder Bioethanol. Von besonderem Interesse sind gegenwärtig Dieselkraftstoffe mit niedrigem Schwefelgehalt, das heißt mit einem Schwefelgehalt von weniger als 0,05 Gew.-%, vorzugsweise von weniger als 0,02 Gew.-%, insbesondere von weniger als 0,005 Gew.-% und speziell von weniger als 0,001 Gew.-% Schwefel. Dieselkraftstoffe können auch Wasser, z. B. in einer Menge bis zu 20 Gew.-%, enthalten, beispielsweise in Form von Diesel-Wasser-Mikroemulsionen oder als sogenannter "White Diesel".

Bei Heizölen handelt es sich beispielsweise um schwefelarme oder schwefelreiche Erdölraffinate oder um Stein- oder Braunkohledestillate, die üblicherweise einen Siedebereich von 150 bis 400°C aufweisen. Bei Heizölen kann es sich um Standard-Heizöl gemäß DIN 51603-1 handeln, das einen Schwefelgehalt von 0,005 bis 0,2 Gew.-% besitzt, oder es handelt sich um schwefelarme Heizöle mit einem Schwefelgehalt von 0 bis 0,005 Gew.-%. Als Beispiele für Heizöl sei insbesondere Heizöl für häusliche Ölfeuerungsanlagen oder Heizöl EL genannt.

Die erfindungsgemäßen Polytetrahydrobenzoxazine oder quaternisierten Polytetrahydrobenzoxazine oder die erfindungsgemäßen Bistetrahydrobenzoxazine können entweder dem jeweiligen Grundkraftstoff, insbesondere dem Otto- oder dem Dieselkraftstoff, allein oder in Form von Kraftstoffadditiv-Paketen, z.B. den sogenannten Diesel-Performance-Paketen, zugesetzt werden. Derartige Pakete stellen Kraftstoffadditiv-Konzentrate dar und enthalten in der Regel neben Lösungsmitteln noch eine Reihe weiterer Komponenten als Coadditive, beispielsweise Trägeröle, Kaltfließverbesserer, Korrosionsinhibitoren, Demulgatoren, Dehazer, Antischaummittel, weitere Cetanzahlverbesserer, weitere Verbrennungsverbesserer, Antioxidantien oder Stabilisatoren, Antistatika, Metallocene, Metalldeaktivatoren, Lösungsvermittler, Marker und/oder Farbstoffe.

In einer bevorzugten Ausführungsform umfasst der additivierte Otto- oder Dieselkraftstoff neben den erfindungsgemäßen Polytetrahydrobenzoxazinen oder quaternisierten Polytetrahydrobenzoxazinen oder erfindungsgemäßen Bistetrahydrobenzoxazinen als weitere Kraftstoffadditive inbesondere mindestens ein (weiteres) Detergensadditiv, nachfolgend als Komponente (D) bezeichnet.

Als Detergentien oder Detergens-Additive (D) werden üblicherweise Ablagerungsinhibitoren für Kraftstoffe bezeichnet. Vorzugsweise handelt es sich bei den Detergentien um amphiphile Substanzen, die mindestens einen hydrophoben Kohlenwasserstoffrest mit einem zahlengemittelten Molekulargewicht (Mₙ) von 85 bis 20.000, insbesondere von 300 bis 5000, vor allem von 500 bis 2500, und mindestens eine polare Gruppierung besitzen, die ausgewählt ist unter
(Da) Mono- oder Polyaminogruppen mit bis zu 6 Stickstoffatomen, wobei mindestens ein Stickstoffatom basische Eigenschaften hat;
(Db) Nitrogruppen, gegebenenfalls in Kombination mit Hydroxylgruppen;
(Dc) Hydroxylgruppen in Kombination mit Mono- oder Polyaminogruppen, wobei min destens ein Stickstoffatom basische Eigenschaften hat;
(Dd) Carboxylgruppen oder deren Alkalimetall- oder Erdalkalimetallsalzen;
(De) Sulfonsäuregruppen oder deren Alkalimetall- oder Erdalkalimetallsalzen;
(Df) Polyoxy-C₂-C₄-alkylengruppierungen, die durch Hydroxylgruppen, Mono- oder Polyaminogruppen, wobei mindestens ein Stickstoffatom basische Eigenschaften hat, oder durch Carbamatgruppen terminiert sind;
(Dg) Carbonsäureestergruppen;
(Dh) aus Bernsteinsäureanhydrid abgeleiteten Gruppierungen mit Hydroxy- und/oder Amino- und/oder Amido- und/oder Imidogruppen; und/oder
(Di) durch Mannich-Umsetzung von substituierten Phenolen mit Aldehyden und Mo no- oder Polyaminen erzeugten Gruppierungen.

Der hydrophobe Kohlenwasserstoffrest in den obigen Detergens-Additiven, welcher für die ausreichende Löslichkeit in der Brennstoffölzusammensetzung sorgt, hat ein zahlengemitteltes Molekulargewicht (Mₙ) von 85 bis 20.000, insbesondere von 300 bis 5000, vor allem von 500 bis 2500. Als typischer hydrophober Kohlenwasserstoffrest, insbesondere in Verbindung mit den polaren Gruppierungen (Da), (Dc), (Dh) und (Di), kommen längerkettige Alkyl- oder Alkenylgruppen, insbesondere der Polypropenyl-, Polybutenyl- und Polyisobutenylrest mit jeweils Mₙ = 300 bis 5000, insbesondere 500 bis 2500, vor allem 700 bis 2300, in Betracht.

Als Beispiele für obige Gruppen von Detergens-Additiven seien die folgenden genannt:
Mono- oder Polyaminogruppen (Da) enthaltende Additive sind vorzugsweise Polyalkenmono- oder Polyalkenpolyamine auf Basis von Polypropen oder konventionellem (d.h. mit überwiegend mittenständigen Doppelbindungen) Polybuten oder Polyisobuten mit Mₙ = 300 bis 5000. Geht man bei der Herstellung der Additive von Polybuten oder Polyisobuten mit überwiegend mittenständigen Doppelbindungen (meist in der β- und γ-Position) aus, bietet sich der Herstellweg durch Chlorierung und anschließende Aminierung oder durch Oxidation der Doppelbindung mit Luft oder Ozon zur Carbonyloder Carboxylverbindung und anschließende Aminierung unter reduktiven (hydrierenden) Bedingungen an. Zur Aminierung können hier Amine, wie z. B. Ammoniak, Monoamine oder Polyamine, wie Dimethylaminopropylamin, Ethylendiamin, Diethylentriamin, Triethylentetramin oder Tetraethylenpentamin, eingesetzt werden. Entsprechende Additive auf Basis von Polypropen sind insbesondere in der WO-A-94/24231 beschrieben.

Weitere bevorzugte Monoaminogruppen (Da) enthaltende Additive sind die Hydrierungsprodukte der Umsetzungsprodukte aus Polyisobutenen mit einem mittleren Polymerisationsgrad P = 5 bis 100 mit Stickoxiden oder Gemischen aus Stickoxiden und Sauerstoff, wie sie insbesondere in WO-A-97/03946 beschrieben sind.

Weitere bevorzugte Monoaminogruppen (Da) enthaltende Additive sind die aus Polyisobutenepoxiden durch Umsetzung mit Aminen und nachfolgender Dehydratisierung und Reduktion der Aminoalkohole erhältlichen Verbindungen, wie sie insbesondere in DE-A-196 20 262 beschrieben sind.

Nitrogruppen (Db), gegebenenfalls in Kombination mit Hydroxylgruppen, enthaltende Additive sind vorzugsweise Umsetzungsprodukte aus Polyisobutenen des mittleren Polymerisationsgrades P = 5 bis 100 oder 10 bis 100 mit Stickoxiden oder Gemischen aus Stickoxiden und Sauerstoff, wie sie insbesondere in WO-A-96/03367 und WO-A-96/03479 beschrieben sind. Diese Umsetzungsprodukte stellen in der Regel Mischungen aus reinen Nitropolyisobutenen (z. B. α,β-Dinitropolyisobuten) und gemischten Hydroxynitropolyisobutenen (z. B. α-Nitro-β-hydroxypolyisobuten) dar.

Hydroxylgruppen in Kombination mit Mono- oder Polyaminogruppen (Dc) enthaltende Additive sind insbesondere Umsetzungsprodukte von Polyisobutenepoxiden, erhältlich aus vorzugsweise überwiegend endständige Doppelbindungen aufweisendem Polyisobuten mit Mₙ = 300 bis 5000, mit Ammoniak, Mono- oder Polyaminen, wie sie insbesondere in EP-A-476 485 beschrieben sind.

Carboxylgruppen oder deren Alkalimetall- oder Erdalkalimetallsalze (Dd) enthaltende Additive sind vorzugsweise Copolymere von C₂-C₄₀-Olefinen mit Maleinsäureanhydrid mit einer Gesamt-Molmasse von 500 bis 20000, deren Carboxylgruppen ganz oder teilweise zu den Alkalimetall- oder Erdalkalimetallsalzen und ein verbleibender Rest der Carboxylgruppen mit Alkoholen oder Aminen umgesetzt sind. Solche Additive sind insbesondere aus der EP-A-307 815 bekannt. Derartige Additive dienen hauptsächlich zur Verhinderung von Ventilsitzverschleiß und können, wie in der WO-A-87/01126 beschrieben, mit Vorteil in Kombination mit üblichen Kraftstoffdetergenzien wie Poly(iso)-butenaminen oder Polyetheraminen eingesetzt werden.

Sulfonsäuregruppen oder deren Alkalimetall- oder Erdalkalimetallsalze (De) enthaltende Additive sind vorzugsweise Alkalimetall- oder Erdalkalimetallsalze eines Sulfobernsteinsäurealkylesters, wie er insbesondere in der EP-A-639 632 beschrieben ist. Derartige Additive dienen hauptsächlich zur Verhinderung von Ventilsitzverschleiß und können mit Vorteil in Kombination mit üblichen Kraftstoffdetergenzien wie Poly(iso)butenaminen oder Polyetheraminen eingesetzt werden.

Polyoxy-C₂-C₄-alkylengruppierungen (Df) enthaltende Additive sind vorzugsweise Polyether oder Polyetheramine, welche durch Umsetzung von C₂-C₆₀-Alkanolen, C₆-C₃₀-Alkandiolen, Mono- oder Di-C₂-C₃₀-alkylaminen, C₁-C₃₀-Alkylcyclohexanolen oder C₁-C₃₀-Alkylphenolen mit 1 bis 30 mol Ethylenoxid und/oder Propylenoxid und/oder Butylenoxid pro Hydroxylgruppe oder Aminogruppe und, im Falle der Polyetheramine, durch anschließende reduktive Aminierung mit Ammoniak, Monoaminen oder Polyaminen erhältlich sind. Derartige Produkte werden insbesondere in EP-A-310 875, EP-A-356 725, EP-A-700 985 und US-A-4 877 416 beschrieben. Im Falle von Polyethern erfüllen solche Produkte auch Trägeröleigenschaften. Typische Beispiele hierfür sind Tridecanol- oder Isotridecanolbutoxylate, Isononylphenolbutoxylate sowie Polyisobutenolbutoxylate und -propoxylate sowie die entsprechenden Umsetzungsprodukte mit Ammoniak.

Carbonsäureestergruppen (Dg) enthaltende Additive sind vorzugsweise Ester aus Mono-, Di- oder Tricarbonsäuren mit langkettigen Alkanolen oder Polyolen, insbesondere solche mit einer Mindestviskosität von 2 mm²/s bei 100°C, wie sie insbesondere in DE-A-38 38 918 beschrieben sind. Als Mono-, Di- oder Tricarbonsäuren können aliphatische oder aromatische Säuren eingesetzt werden, als Esteralkohole bzw. -polyole eignen sich vor allem langkettige Vertreter mit beispielsweise 6 bis 24 C-Atomen. Typische Vertreter der Ester sind Adipate, Phthalate, iso-Phthalate, Terephthalate und Trimellitate des iso-Octanols, iso-Nonanols, iso-Decanols und des iso-Tridecanols. Derartige Produkte erfüllen auch Trägeröleigenschaften.

Aus Bernsteinsäureanhydrid abgeleitete Gruppierungen mit Hydroxy- und/oder Amino- und/oder Amido- und/oder Imidogruppen (Dh) enthaltende Additive sind vorzugsweise entsprechende Derivate von Alkyl- oder Alkenyl-substituiertem Bernsteinsäureanhydrid und insbesondere die entsprechenden Derivate von Polyisobutenylbernsteinsäureanhydrid, welche durch Umsetzung von konventionellem oder hochreaktivem Polyisobuten mit Mₙ = 300 bis 5000 mit Maleinsäureanhydrid auf thermischem Weg oder über das chlorierte Polyisobuten erhältlich sind. Von besonderem Interesse sind hierbei Derivate mit aliphatischen Polyaminen wie Ethylendiamin, Diethylentriamin, Triethylentetramin oder Tetraethylenpentamin. Bei den Gruppierungen mit Hydroxy- und/oder Amino- und/oder Amido- und/oder Imidogruppen handelt es sich beispielsweise um Carbonsäuregruppen, Säureamide von Monoaminen, Säureamide von Di- oder Polyaminen, die neben der Amidfunktion noch freie Amingruppen aufweisen, Bernsteinsäurederivate mit einer Säure- und einer Amidfunktion, Carbonsäureimide mit Monoaminen, Carbonsäureimide mit Di- oder Polyaminen, die neben der Imidfunktion noch freie Amingruppen aufweisen, oder Diimide, die durch die Umsetzung von Di- oder Polyaminen mit zwei Bernsteinsäurederivaten gebildet werden. Derartige Kraftstoffadditive sind insbesondere in US-A-4 849 572 beschrieben.

Bei den Detergens-Additiven aus der Gruppe (Dh) handelt es sich vorzugsweise um die Umsetzungsprodukte von Alkyl- oder Alkenyl-substituierten Bernsteinsäureanhydriden, insbesondere von Polyisobutenylbernsteinsäureanhydriden, mit Aminen und/oder Alkoholen. Es handelt sich hierbei somit um von Alkyl-, Alkenyl- oder Polyisobutenyl-Bernsteinsäureanhydrid abgeleitete Derivate mit Amino- und/oder Amido- und/oder Imido- und/oder Hydroxylgruppen. Es versteht sich von selbst, dass diese Umsetzungsprodukte nicht nur bei Einsatz von substituiertem Bernsteinsäureanhydrid, sondern auch bei Verwendung von substituierter Bernsteinsäure oder geeigneten Säurederivaten, wie Bernsteinsäurehalogenide oder -ester, erhältlich sind.

Vorzugsweise umfasst der additivierte Kraftstoff mindestens ein Detergens auf Basis eines Polyisobutenyl-substituierten Bernsteinsäureimids. Speziell von Interesse sind die Imide mit aliphatischen Polyaminen. Besonders bevorzugte Polyamine sind dabei Ethylendiamin, Diethylentriamin, Triethylentetramin, Pentaethylenhexamin und vor allem Tetraethylenpentamin. Der Polyisobutenylrest besitzt ein zahlenmittleres Molekulargewicht Mₙ von vorzugsweise 500 bis 5000, besonders bevorzugt von 500 bis 2000 und insbesondere von etwa 1000.

Durch Mannich-Umsetzung von substituierten Phenolen mit Aldehyden und Mono- oder Polyaminen erzeugte Gruppierungen (Di) enthaltende Additive sind vorzugsweise Umsetzungsprodukte von Polyisobuten-substituierten Phenolen mit Formaldehyd und Mono- oder Polyaminen wie Ethylendiamin, Diethylentriamin, Triethylentetramin, Tetraethylenpentamin oder Dimethylaminopropylamin. Die Polyisobutenyl-substituierten Phenole können aus konventionellem oder hochreaktivem Polyisobuten mit Mₙ = 300 bis 5000 stammen. Derartige "Polyisobuten-Mannichbasen" sind insbesondere in der EP-A-831 141 beschrieben.

Vorzugsweise werden die genannten Detergens-Additive (D) zusammen mit den erfindungsgemäßen Polytetrahydrobenzoxazinen oder quaternisierten Polytetrahydrobenzoxazinen oder den erfindungsgemäßen Bistetrahydrobenzoxazinen in Kombination mit wenigstens einem Trägeröl verwendet.

Geeignete mineralische Trägeröle sind bei der Erdölverarbeitung anfallende Fraktionen, wie Brightstock oder Grundöle mit Viskositäten wie beispielsweise aus der Klasse SN 500 - 2000; aber auch aromatische Kohlenwasserstoffe, paraffinische Kohlenwasserstoffe und Alkoxyalkanole. Brauchbar ist ebenfalls eine als "hydrocrack oil" bekannte und bei der Raffination von Mineralöl anfallende Fraktion (Vakuumdestillatschnitt mit einem Siedebereich von etwa 360 bis 500°C, erhältlich aus unter Hochdruck katalytisch hydriertem und isomerisiertem sowie entparaffiniertem natürlichen Mineralöl). Ebenfalls geeignet sind Mischungen oben genannter mineralischer Trägeröle.

Beispiele für geeignete synthetische Trägeröle sind ausgewählt unter: Polyolefinen (Polyalphaolefinen oder Polyinternalolefinen), (Poly)estern, (Poly)alkoxylaten, Polyethern, aliphatischen Polyetheraminen, alkylphenolgestarteten Polyethern, alkylphenolgestarteten Polyetheraminen und Carbonsäureestern langkettiger Alkanole.

Beispiele für geeignete Polyolefine sind Olefinpolymerisate mit Mₙ = 400 bis 1800, vor allem auf Polybuten- oder Polyisobuten-Basis (hydriert oder nicht hydriert).

Beispiele für geeignete Polyether oder Polyetheramine sind vorzugsweise Polyoxy-C₂-C₄-alkylengruppierungen enthaltende Verbindungen, welche durch Umsetzung von C₂-C₆₀-Alkanolen, C₆-C₃₀-Alkandiolen, Mono- oder Di-C₂-C₃₀-alkylaminen, C₁-C₃₀-Alkyl-cyclohexanolen oder C₁-C₃₀-Alkylphenolen mit 1 bis 30 mol Ethylenoxid und/ oder Propylenoxid und/oder Butylenoxid pro Hydroxylgruppe oder Aminogruppe und, im Falle der Polyetheramine, durch anschließende reduktive Aminierung mit Ammoniak, Monoaminen oder Polyaminen erhältlich sind. Derartige Produkte werden insbesondere in EP-A 310 875, EP-A 356 725, EP-A 700 985 und US-A 4,877,416 beschrieben. Beispielsweise können als Polyetheramine Poly-C₂-C₆-Alkylenoxidamine oder funktionelle Derivate davon verwendet werden. Typische Beispiele hierfür sind Tridecanol- oder Isotridecanolbutoxylate, Isononylphenolbutoxylate sowie Polyisobutenolbutoxylate und -propoxylate sowie die entsprechenden Umsetzungsprodukte mit Ammoniak.

Beispiele für Carbonsäureester langkettiger Alkanole sind insbesondere Ester aus Mono-, Di- oder Tricarbonsäuren mit langkettigen Alkanolen oder Polyolen , wie sie insbesondere in der DE-A 38 38 918 beschrieben sind. Als Mono-, Di- oder Tricarbonsäuren können aliphatische oder aromatische Säuren eingesetzt werden, als Esteralkohole bzw. -polyole eignen sich vor allem langkettige Vertreter mit beispielsweise 6 bis 24 C-Atomen. Typische Vertreter der Ester sind Adipate, Phthalate, iso-Phthalate, Terephthalate und Trimellitate des Isooctanols, Isononanols, Isodecanols und des Isotridecanols, wie z. B. Di-(n- oder iso-tridecyl)phthalat.

Weitere geeignete Trägerölsysteme sind beispielsweise in DE-A 38 26 608, DE-A 41 42 241, DE-A 43 09 074, EP-A 0 452 328 und EP-A 0 548 617 beschrieben.

Beispiele für besonders geeignete synthetische Trägeröle sind alkoholgestartete Polyether mit etwa 5 bis 35, wie z. B. etwa 5 bis 30, C₃-C₆-Alkylenoxideinheiten, wie z. B. ausgewählt unter Propylenoxid-, n-Butylenoxid- und i-Butylenoxid-Einheiten, oder Gemischen davon. Nichtlimitierende Beispiele für geeignete Starteralkohole sind langkettige Alkanole oder mit langkettigem Alkyl-substituierte Phenole, wobei der langkettige Alkylrest insbesondere für einen geradkettigen oder verzweigten C₆-C₁₈-Alkylrest steht. Als bevorzugte Beispiele sind zu nennen Tridecanol und Nonylphenol.

Weitere geeignete synthetische Trägeröle sind alkoxylierte Alkylphenole, wie sie in der DE-A 101 02 913 beschrieben sind.

Bevorzugte Trägeröle sind synthetische Trägeröle, wobei Polyether besonders bevorzugt sind.

Die erfindungsgemäße Kraftstoffzusammensetzung enthält die erfindungsgemäßen Polytetrahydrobenzoxazine oder quaternisierten Polytetrahydrobenzoxazine oder die erfindungsgemäßen Bistetrahydrobenzoxazine in einer Menge von üblicherweise 10 bis 2000 Gew.-ppm, besonders bevorzugt von 20 bis 1000 Gew.-ppm, stärker bevorzugt von 30 bis 500 Gew.-ppm und insbesondere von 40 bis 200 Gew.-ppm, z. B. von 50 bis 150 Gew.-ppm.

Werden die erfindungsgemäßen Polytetrahydrobenzoxazine oder quaternisierten Polytetrahydrobenzoxazine oder die erfindungsgemäßen Bis-tetrahydrobenzoxazine dem Kraftstoff in Kombination mit einem oder mehreren (weiteren) Detergensadditiven aus der Gruppe (D) zugesetzt, beträgt die Gesamtmenge an diesen beiden Additivsorten üblicherweise 10 bis 3000 Gew.-ppm, besonders bevorzugt von 20 bis 1500 Gew.-ppm, stärker bevorzugt von 30 bis 1000 Gew.-ppm und insbesondere von 40 bis 500 Gew.-ppm, z.B. von 50 bis 300 Gew.-ppm.

Wenn ein Trägeröl mitverwendet wird, so wird dieses dem erfindungsgemäßen additivierten Kraftstoff in einer Menge von vorzugsweise 1 bis 1000 Gew.-ppm, besonders bevorzugt von 10 bis 500 Gew.-ppm und insbesondere von 20 bis 100 Gew.-ppm, zugesetzt.

Als weitere Coadditive geeignete Kaltfließverbesserer sind beispielsweise Copolymere von Ethylen mit wenigstens einem weiteren ungesättigten Monomer, z.B. EthylenVinylacetat-Copolymere.

Als weitere Coadditive geeignete Korrosionsinhibitoren sind beispielsweise Bernsteinsäureester, vor allem mit Polyolen, Fettsäurederivate, z.B. Ölsäureester, oligomerisierte Fettsäuren und substituierte Ethanolamine.

Als weitere Coadditive geeignete Demulgatoren sind beispielsweise die Alkali- und Erdalkalimetallsalze von alkylsubstituierten Phenol- und Naphthalinsulfonaten und die Alkali- und Erdalkalimetallsalze von Fettsäure, weiterhin Alkoholalkoxylate, z.B. Alkoholethoxylate, Phenolalkoxylate, z.B. tert.-Butylphenolethoxylate oder tert.-Pentyl-phenolethoxylate, Fettsäure, Alkylphenole, Kondensationsprodukte von Ethylenoxid und Propylenoxid, z.B. Ethylenoxid-Propylenoxid-Blockcopolymere, Polyethylenimine und Polysiloxane.

Als weitere Coadditive geeignete Dehazer sind beispielsweise alkoxylierte Phenol-Formaldehyd-Kondensate.

Als weitere Coadditive geeignete Antischaummittel sind beispielsweise Polyethermodifizierte Polysiloxane.

Als weitere Coadditive geeignete Cetanzahl- und Verbrennungsverbesserer sind beispielsweise Alkylnitrate, z.B. Cyclohexylnitrat und insbesondere 2-Ethylhexylnitrat, und Peroxide, z.B. Di-tert.-butylperoxid.

Als weitere Coadditive geeignete Antioxidantien sind beispielsweise substituierte Phenole, z.B. 2,6-Di-tert.-butylphenol und 2,6-Di-tert.-butyl-3-methylphenol, sowie Phenylendiamine, z.B. N,N'-Di-sec.-butyl-p-phenylendiamin.

Als weitere Coadditive geeignete Metalldeakivatoren sind beispielsweise Salicylsäure-Derivate, z.B. N,N'-Disalicyliden-1,2-propandiamin.

Als Lösungsmittel eignen sich, insbesondere für Kraftstoffadditiv-Pakete, beispielsweise unpolare organische Lösungsmittel, insbesondere aromatische und aliphatische Kohlenwasserstoffe, z.B. Toluol, Xylole, "white spirit" sowie die technischen Lösungsmittelgemische der Bezeichnungen Shellsol® (Hersteller: Royal Dutch / Shell Group), Exxol® (Hersteller: ExxonMobil) und Solvent Naphtha. Weiterhin kommen hier, insbesondere in Abmischung mit den genannten unpolaren organischen Lösungsmitteln, polare organische Lösungsmittel, vor allem Alkohole wie 2-Ethylhexanol, Decanol und Isotridecanol, in Betracht.

Wenn die genannten Coadditive und/oder Lösungsmittel in Otto- oder Dieselkraftstoff mitverwendet werden, werden sie in den hierfür übliche Mengen eingesetzt.

Die erfindungsgemäßen Polytetrahydrobenzoxazine und quaternisierten Polytetrahydrobenzoxazine und die erfindungsgemäßen Bistetrahydrobenzoxazine eignen sich weiterhin in besonders vorteilhafter Weise als Schmierstoffadditiv. Als Schmierstoffe oder Schmierstoffzusammensetzungen sollen hier Motorenöle, Schmieröle, Getriebe-, Schalt- und Automatiköle und verwandte flüssige Zusammensetzungen, die der Schmierung von mechanisch bewegten Teilen - zumeist als Metall - dienen, bezeichnet werden. Die erfindungsgemäßen Polytetrahydrobenzoxazine und quaternisierten Polytetrahydrobenzoxazine und die erfindungsgemäßen Bistetrahydrobenzoxazine wirken in den Schmierstoffzusammensetzungen hauptsächlich als Dispergatoradditiv und/oder als Detergensadditiv.

Die erfindungsgemäße Schmierstoffzusammensetzung enthält die erfindungsgemäßen Polytetrahydrobenzoxazine oder quaternisierten Polytetrahydrobenzoxazine oder die erfindungsgemäßen Bistetrahydrobenzoxazine in einer Menge von üblicherweise 0,001 bis 20 Gew.-%, vorzugsweise 0,01 bis 10 Gew.-%, insbesondere 0,05 bis 8 Gew.-% und vor allem 0,1 bis 5 Gew.-%, bezogen auf die Gesamtmenge der Schmierstoffzusammensetzung.

Die wirtschaftlich bedeutsamsten Schmierstoffzusammensetzungen sind Motorenöle sowie Getriebe-, Schalt- und Automatiköle. Motorenöle bestehen üblicherweise aus mineralischen Grundölen, welche überwiegend paraffinische Bestandteile enthalten und durch aufwendige Aufarbeitungs- und Reinigungsprozesse in der Raffinerie hergestellt werden, mit einem Anteil an ca. 2 bis 10 Gew.-% an Additiven (bezogen auf die Wirksubstanz-Gehalte). Für spezielle Anwendungen, beispielsweise Hochtemperatur-Einsätze, können die mineralischen Grundöle teilweise oder vollständig durch synthetische Komponenten wie organische Ester, synthetische Kohlenwasserstoffe wie Olefinoligomere, Poly-α-Olefine oder Polyolefine oder Hydrocrack-Öle ersetzt sein. Motorenöle müssen auch bei hohen Temperaturen ausreichend hohe Viskositäten aufweisen, um einen einwandfreien Schmiereffekt und eine gute Abdichtung zwischen Zylinder und Kolben zu gewährleisten. Weiterhin müssen Motorenöle von ihren Fließeigenschaften auch so beschaffen sein, dass bei niedrigen Temperaturen der Motor problemlos gestartet werden kann. Motorenöle müssen oxidationsstabil sein und dürfen auch unter schweren Arbeitsbedingungen nur wenig Zersetzungsprodukte in flüssiger oder fester Form sowie Ablagerungen erzeugen. Motorenöle dispergieren Feststoffe (Dispersant-Verhalten), verhindern Ablagerungen (Detergent-Verhalten), neutralisieren saure Reaktionsprodukte und bilden einen Verschleißschutzfilm auf den Metalloberflächen im Motor aus. Motorenöle werden üblicherweise nach Viskositätsklassen-Klassen (SAE-Klassen) charakterisiert.

Getriebe-, Schalt- und Automatiköle sind bezügliche ihrer Grundkomponenten und Additive ähnlich wie Motorenöle zusammengesetzt. Die Kraftübertragung im Zahnradsystem von Getrieben erfolgt zu einem hohen Anteil durch den Flüssigkeitsdruck im Getriebeöl zwischen den Zähnen. Das Getriebeöl muß demzufolge so beschaffen sein, dass es auf Dauer hohe Drücke aushält, ohne sich zu zersetzen. Neben den Viskositätseigenschaften sind hier Verschleiß, Druckfestigkeit, Reibung, Scherstabilität, Traktion und Einlaufverhalten die entscheidenden Größen.

Motorenöle und Getriebe-, Schalt- und Automatiköle enthalten neben den erfindungsgemäßen Polytetrahydrobenzoxazinen oder quaternisierten Polytetrahydrobenzoxazinen oder den erfindungsgemäßen Bistetrahydrobenzoxazinen in der Regel noch mindestens eines, meist jedoch einige oder alle der nachfolgend aufgeführten Additive in den hierfür in der Regel üblichen Mengen (welche in Gew.-%, bezogen auf die Gesamt-Schmierstoffzusammensetzung, in Klammern angegeben sind):
- Antioxidantien (0,1 bis 5 %):
   Schwefelverbindungen, z.B. Reaktionsprodukte von Terpenen (α-Pinen), Harzölen oder niedermolekularen Polybutenen mit Schwefel, Dialkylsulfide, Dialkyltrisulfide, Polysulfide, Diarylsulfide, modifizierte Thiole, Mercaptobenzimidazole, Mercaptotriazine, Thiophen-Derivate, Xanthate, Zink-dialkyldithiocarbamate, Thioglykole, Thioaldehyde, Dibenzyldisulfid, Alkylphenolsulfide, Dialkylphenolsulfide oder schwefelhaltige Carbonsäuren
   Phosphorverbindungen, z.B. Triaryl- und Trialkylphosphite, 3,5-Di-tert.-butyl-4-hydroxybenzyl-phosphonsäure-dialkylester oder Phosphonsäurepiperazide
   Schwefel-Phosphor-Verbindungen, z.B. Zink-dialkyldithiophosphate (Metalldialkyldithiophosphate wirken in Schmierölen auch als Korrosionsinhibitoren und Hochdruckadditive) oder Reaktionsprodukte von Phosphorpentasulfid mit Terpenen (α-Pinen, Dipenten), Polybutenen, Olefinen oder ungesättigten Estern
   Phenol-Derivate, z.B. sterisch gehinderte Mono-, Bis- oder Trisphenole, sterisch gehinderte mehrkernige Phenole, Polyalkylphenole, 2,6-Di-tert.-butyl-4-methylphenol oder Methylen-4,4'-bis(2,6-di-tert.-butylphenol) (Phenol-Derivate werden oft in Kombination mit Antioxidantien auf Schwefelbasis oder Aminbasis eingesetzt) Amine, z.B. Aryl-amine wie Diphenylamin, Phenyl-α-naphthylamin oder 4,4'-Tetramethyldiamino-diphenylmethan
   Metalldeaktivatoren im engeren Sinne, z.B. N-Salicyliden-ethylamin, N,N'-Disalicyliden-ethylendiamin, N,N'-Disalicyliden-1,2-propandiamin, Triethylendiamin, Ethylendiamintetraessigsäure, Phosphorsäure, Zitronensäure, Glykolsäure, Lecithin, Thiadiazol, Imidazol oder Pyrazol-Derivate
- Viskositätsindex-Verbesserer (0,05 bis 10 %), z.B: Polyisobutene mit einem Molekulargewicht von üblicherweise 10.000 bis 45.000, Polymethacrylate mit einem Molekulargewicht von üblicherweise15.000 bis 100.000, Homo- und Copolymerisate von 1,3-Dienen wie Butadien oder Isopren mit einem Molekulargewicht von üblicherweise 80.000 bis 100.000, 1,3-Dien-Styrol-Copolymerisate mit einem Molekulargewicht von üblicherweise 80.000 bis 100.000, Maleinsäureanydrid-StyrolPolymere in veresterter Form mit einem Molekulargewicht von üblicherweise 60.000 bis 120.000, sternförmige Polymere mit blockförmigem Aufbau durch Einheiten aus konjugierten Dienen und aromatischen Monomeren mit einem Molekulargewicht von üblicherweise 200.000 bis 500.000, Polyalkylstyrole mit einem Molekulargewicht von üblicherweise 80.000 bis 150.000, Polyolefine aus Ethylen und Propylen oder Styrol-Cyclopentadien-Norbornen-Terpolymere mit einem Molekulargewicht von üblicherweise 60.000 bis 140.000
- Pour Point Erniedriger (Kaltfließverbesserer) (0,03 bis 1 %), z.B. bicyclische Aromaten wie Naphthalin mit verschiedenen langkettigen Alkylresten, Polymethyacrylate mit 12 bis 18 Kohlenstoffatomen im Alkoholrest, einem Verzweigungsgrad zwischen 10 bis 30 mol-% und einem durchschnittlichen Molekulargewicht von 5.000 bis 500.000, langkettige Alkylphenole und Phthalsäure-dialkylarylester oder Copolymere verschiedener Olefine
- Detergentien (HD Additive) (0,2 bis 4 %), z.B. Calcium-, Blei-, Zink- und Mangan-Naphthenate, Calcium-Dichlorostearate, Calcium-Phenylstearate, Calcium-Chlorophenylstearate, Sulfonierungsprodukte von Alkylaromaten wie Dodecylbenzol, Petroleumsulfonate, Natrium-, Calcium-, Barium- oder Magnesium-Sulfonate, neutrale, basische und überbasische Sulfonate, Phenate und Carboxylate, Salicylate, Metallsalze von Alkylphenolen und Alkylphenolsulfiden, Phosphate, Thiophosphate oder Alkenylphosphonsäure-Derivate
- Aschefreie Dispersantien (0,5 bis 10 %), z.B. Mannich-Kondensate aus Alkylphenol, Formaldehyd und Polyalkylenpolyaminen, Umsetzungsprodukte von Polyisobutenylsuccinanhydriden mit Polyhydroxy-Verbindungen oder Polyaminen, Copolymerisate von Alkylmethacrylaten mit Diethylaminoethyl-methacrylat, N-Vinylpyrrolidon, N-Vinyl-pryridin oder 2-Hydroxyethyl-methacrylat oder Vinylacetat-Fumarat-Copoly-merisate
- Hochdruckadditive (Extreme Pressure Additive) (0,2 bis 2,5 %), z.B. chlorierte Paraffine mit 40 bis 70 Gew.-% Chlorgehalt, chlorierte Fettsäure (insbesondere mit Trichlormethyl-Endgruppen), Dialkylhydrogenphosphite, Triarylphosphite, Arylphosphate wie Trikresylphosphat, Dialkylphosphate, Trialkylphosphate wie Tributylphosphat, Trialkylphosphine, Diphosphorsäureester, Nitroaromaten, Aminophenol-Derivate der Naphthensäure, Carbaminsäureester, Dithiocarbaminsäure-Derivate, substituierte 1,2,3-Triazole, Mischungen aus Benzotriazol und Alkylbernsteinsäureanhydrid oder Alkylmaleinsäureanhydrid, 1,2,4-Thiadiazol-Polymere, Morpholinobenzothiadiazol-disulfid, chlorierte Alkylsulfide, sulfurisierte Olefine, sulfurisierte Chlornaphthaline, chlorierte Alkylthiocarbonate, organische Sulfide und Polysulfide wie Bis-(4-chlorbenzyl)-disulfid und Tetrachlordiphenylsulfid, Trichloracrolein-Mercaptale oder insbesondere Zinkdialkyldithiophosphate (ZDDP)
- Reibungsverminderer (Friction Modifier) (0,05 bis 1 %), insbesondere polare öllösliche Verbindungen, die eine dünne Schicht auf der Reibungsfläche durch Adsorption erzeugen, z.B. Fettalkohole, Fettamide, Fettsäuresalze, Fettsäurealkylester oder Fettsäureglyceride
- Antischaum-Additive (0,0001 bis 0,2 %), z.B. flüssige Silicone wie Polydimethylsiloxane oder Polhethylenglykolether und -sulfide
- Demulgatoren (0,1 bis 1 %), z.B. Dinonylnaphthalinsulfonate in Form ihrer Alkali- und Erdalkalimetallsalze
- Korrosionsinhibitoren (auch als Metalldeaktivatoren bezeichnet) (0,01 bis 2 %), z.B. tertiäre Amine und ihre Salze, Iminoester, Amidoxime, Diaminomethane, Derivate von gesättigten oder ungesättigten Fettsäuren mit Alkanolaminen, Alkylamine, Sarcosine, Imidazoline, Alkylbenzotriazole, Dimercaptothiadiazol-Derviate, Diarylphosphate, Thiophosphorsäureester, Neutralsalze von primären n-C₈-C₁₈-Alkylaminen oder Cycloalkylaminen mit Dialkylphosphaten mit verzweigten C₅-C₁₂-Alkylgruppen, neutrale oder basische Erdalkalimetallsulfonate, Zinknaphthenate, Mono- und Dialkylarylsulfonate, Barium-dinonylnaphthalinsulfonate, Lanolin (Wollfett), Schwermetallsalze der Naphthensäure, Dicarbonsäure, ungesättigte Fettsäuren, Hydroxyfettsäuren, Fettsäureester, Pentaerythritol- und Sorbitan-Monooleate, O-Stearoylalkanolamine, Polyisobutenylbernsteinsäure-Derivate oder Zinkdialkyldithiophosphate und Zinkdialkyldithiocarbamate
- Emulgatoren (0,01 bis 1 %), z.B. langkettige ungesättigte, natürlich vorkommmende Carbonsäure, Naphthensäuren, synthetische Carbonsäure, Sulfonamide, N-Oleylsarcosin, Alkansulfamidoessigsäure, Dodecylbenzolsulfonat, langkettige alkylierte Ammoniumsalze wie Dimethyldodecylbenzylammoniumchlorid, Imidazolinium-Salze, Alkyl-, Alkylaryl-, Acyl-, Alkylamino- und Acylaminopolyglykole oder langkettige acylierte Mono- und Diethanolamine
- Farbstoffe und Fluoreszenzadditive (0,001 bis 0,2 %)
- Konservierungsmittel (0,001 bis 0,5 %)
- Geruchsverbesserer (0,001 bis 0,2 %)

Typische gebrauchsfertige Motorenöl- und Getriebe-, Schalt- und Automatiköl-Zusammensetzungen im Rahmen der vorliegenden Erfindung sind folgendermaßen zusammengesetzt, wobei sich die Angaben für die Additive auf die Wirksubstanz-Gehalte beziehen und die Summe aller Komponenten immer 100 Gew.-% ergibt:
- 80 bis 99,3 Gew.-%, insbesondere 90 bis 98 Gew.-% Motorenöl- bzw. Getriebe-, Schalt- und Automatiköl-Grundlage (mineralische Grundöle und/oder synthetische Komponenten) einschließlich der Anteile an Lösungs- und Verdünnungsmittel für die Additive
- 0,1 bis 8 Gew.-% erfindungsgemäße Polytetrahydrobenzoxazine oder quaternisierte Polytetrahydrobenzoxazine oder erfindungsgemäße Bistetrahydrobenzoxazine
- 0,2 bis 4 Gew.-%, insbesondere 1,3 bis 2,5 Gew.-% Detergentien der Gruppe (d)
- 0,5 bis 10 Gew.-%, insbesondere 1,3 bis 6,5 Gew.-% Dispersantien der Gruppe (e)
- 0,1 bis 5 Gew.-% ,insbesondere 0,4 bis 2,0 Gew.-% Antioxidantien der Gruppe (a) und/oder Hochdruckadditive der Gruppe (f) und/oder Reibungsverminderer der Gruppe (g)
- 0,05 bis 10 Gew.-%, insbesondere 0,2 bis 1,0 Gew.-% Viskositätsindex-Verbesserer der Gruppe (b)
- 0 bis 2 Gew.-% der sonstigen Additive der Gruppen (c) und (h) bis (n)

Die Erfindung wir anhand der folgenden, nicht einschränkenden Beispiele näher erläutert.

### Herstellungsbeispiele

### Beispiel 1 [Reaktionsschritt (A)]

250 g (3,37 mol) Isobutanol und 60 g (2,0 mol) Paraformaldehyd wurden bei 20°C in 250 g Cyclohexan suspendiert. Anschließend erfolgte die Zugabe von 30 g (0,50 mol) 1,2-Ethylendiamin. Die Reaktionsmischung wurde im Vakuum bei 83 mbar für 5 Stunden bei 40°C unter Rückfluss erhitzt. Abgespaltenes Wasser wurde azeotrop aus dem Reaktionsgemisch entfernt. Aus der Menge an abgespaltenem Wasser wurde ein Umsatz von 75 % bestimmt. Nach Abdestillieren des Lösungsmittels Cyclohexan und des überschüssigen Isobutanols wurde eine Produktmischung mit den Verbindungen IIIa, IIIb und IIIc (im Gew.-Verhältnis von 1 : 0,7 : 0,4) als Hauptprodukte erhalten:

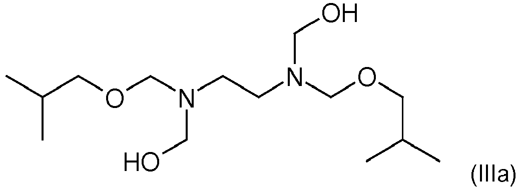

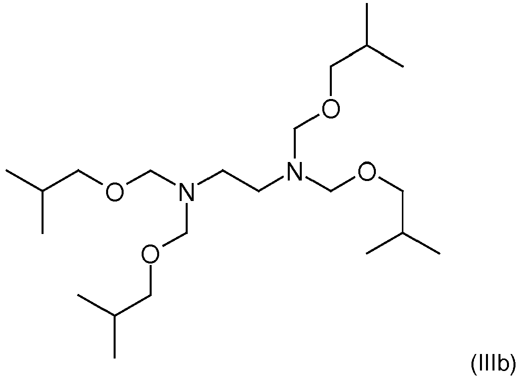

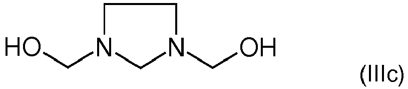

In untergeordneten Mengen liegen in dieser Produktmischung auch noch das entsprechende nur an einer Hydroxymethyl-Gruppierung mit Isobutanol veretherte Tetrakis-(hydroxymethyl)-ethylendiamin und das entsprechende an nur einer Hydroxymethyl-Gruppierung mit Isobutanol veretherte Tris-(hydroxymethyl)-ethylendiamin vor.

Diese Produktmischung zeigte im ¹H-NMR-Spektrum (CDCl₃, δ in ppm) die folgenden wesentlichen Signale:
für IIIa: 0,9 (m, -CH₃), 1,83 (m, -CH-(CH₃)₂), 2,9 (s, -NCH₂CH₂N-), 3,25 (d, -OCH₂-) und 4,18(s, -NCH₂O-);
für IIIb: 0,9 (m, -CH₃), 1,83 (m, -CH-(CH₃)₂), 3,0 (s, -NCH₂CH₂N-), 3,25 (d, -OCH₂-) und 4,3 (s, -NCH₂O-);
für IIIc: 3,7 (s, -NCH₂CH₂N-) und 3,8 (s, -NCH₂N-).

### Beispiel 2 [Reaktionsschritt (B)]

Die in Beispiel 1 hergestellten Produktmischung wurde zusammen mit 262 g (1,0 mol) 4-n-Dodecylphenol in 525 g Solvesso™ 150 gelöst und 2 Stunden auf 90°C erhitzt. Anschließend wurde das Lösungsmittel im Vakuum abdestilliert. Es resultierte ein Bistetrahydrobenzoxazin der Formel IIa (R¹ = n-Dodecyl), welches im ¹H-NMR-Spektrum (CDCl₃, δ in ppm) die folgenden Signale aufwies:
0,5-1,6 (m, -(CH₂)₁₁CH₃), 3,0 (s, -NCH₂CH₂N-), 4,0 (s, -NCH₂-), 4,9 (s, -OCH₂N-), 6,7 (s, aromat. CH, meta-Stellung), 6,8 und 7,0 (d, aromat. CH, ortho- und meta-Stellung).

### Beispiel 3 [Reaktionsschritt (B)]

Die in Beispiel 1 hergestellten Produktmischung wurde zusammen mit 265 g (1,28 mol) 4-tert.-Octylphenol in 520 g Toluol gelöst und 2 Stunden auf 40°C erhitzt. Anschließend wurde das Lösungsmittel im Vakuum abdestilliert. Es resultierte ein Bistetrahydrobenzoxazin der Formel IIa (R¹ = tert.-Octyl), welches im ¹H-NMR-Spektrum (CDCl₃, δ in ppm) die folgenden Signale aufwies:
1,3 (s, CH₃), 1,7 (s, -CH₂-), 3,0 (s, -NCH₂CH₂N-), 4,0 (s, -NCH₂-), 4,9 (s, -OCH₂N-), 6,9 (s, aromat. CH, meta-Stellung), 7,1-7,3 (m, aromat. CH, ortho- und meta-Stellung).

### Beispiel 4 [Reaktionsschritt (B)]

Die in Beispiel 1 hergestellten Produktmischung wurde zusammen mit 281 g (1,29 mol) 4-n-Nonylphenol in 520 g Toluol gelöst und 2 Stunden auf 40°C erhitzt. Anschließend wurde das Lösungsmittel im Vakuum abdestilliert. Es resultierte ein Bistetrahydrobenzoxazin der Formel IIa (R¹ = n-Nonyl), welches im ¹H-NMR-Spektrum (CDCl₃, δ in ppm) die folgenden Signale aufwies:
0,5-1,7 (m, -(CH₂)₈CH₃), 3,0 (s, -NCH₂CH₂N-), 4,0 (s, -NCH₂-), 4,9 (s, -OCH₂N-), 6,7 (s, aromat. CH, meta-Stellung), 6,8 und 7,0 (d, aromat. CH, ortho- und meta-Stellung).

### Beispiel 5 [Reaktionsschritt (C)]

Das Bistetrahydrobenzoxazin aus Beispiel 2 wurde im Gew.-Verhältnis von 1 : 1 in Solvesso^{™} 150 gelöst und 1 Stunde auf 205°C erhitzt. Nach Abdestillieren des Lösungsmittels im Vakuum resultierte ein Polytetrahydrobenzoxazin der Formel la (R¹ = n-Dodecyl, n = ca. 6), welches ein gewichtsmittleres Molekulargewicht (M_{w}) von 4600 g/mol, ein zahlenmittleres Molekulargewicht (Mₙ) von 1500 g/mol und einen Polydispersitätsindex (PDI) von 3,07 aufwies und im ¹H-NMR-Spektrum (CDCl₃, δ in ppm) die folgenden Signale zeigte.
0,5-1,6 (m, -(CH₂)₁₁CH₃), 3,0 (s, -NCH₂CH₂N-), 4,0 (s, -NCH₂-), 4,9 (s, -OCH₂N-), 6,7 (s, aromat. CH, meta-Stellung), 6,8 und 7,0 (d, aromat. CH, meta- und ortho-Stellung).

### Anwendungsbeispiel

Zur Untersuchung des Einflusses der beschriebenen Verbindungen auf die Performance von mit der erfindungsgemäßen Kraftstoffzusammensetzung betriebene direkteinspritzende Dieselmotoren wurde der Leistungsverlust (powerloss) in Anlehnung an die offizielle Testmethode CEC F-98-08 bestimmt. Der Leistungsverlust ist ein direktes Maß für Bildung von Ablagerungen in den Injektoren.

Verwendet wurde ein gebräuchlicher direkteinspritzender Dieselmotor mit Common-Rail-System. Zur ökonomischeren Abwicklung der Bestimmungen wurde gegenüber der CEC F-98-08 ein verkürzter Motorlaufzyklus gewählt, d.h. einmal 12 Stunden Laufzeit gegenüber viermal 8 Stunden Laufzeit unterbrochen von dreimal 8 Stunden Stillstandzeit der ursprünglichen Testvorschrift. Weiterhin wurden bereits eingefahrene und gereinigte Injektoren verwendet. Allen anderen Testdetails wurden wie in der CEC F-98-08 erfüllt.

Als Kraftstoff wurde ein handelsüblicher Dieselkraftstoff der Fa. Haltermann (RF-06-03 Batch 12) eingesetzt. Diesem wurden zur künstlichen Anregung der Bildung von Ablagerungen an den Injektoren 2 Gew.-ppm Zink-Didodecanoat zugesetzt.

Als Additiv wurde die Verbindung aus Beispiel 5 verwendet.

Die nachfolgende Tabelle zeigt die Ergebnisse der powerloss-Bestimmungen:

| Prüflauf-Nr. | Detergens-Additiv | Dosierung [Gew.-ppm Wirksubstanz] | powerloss, 12 h [bei 4000 rpm] |
|---|---|---|---|
| Grundwert | ohne | - | 4,34 % |
| 1 | Beispiel 5 | 150 | 0.41 % |
| 2 | Beispiel 5 | 150 | 0.71 % |

## Patentansprüche

1. Polytetrahydrobenzoxazine, erhältlich durch die Reaktionsschritte
(A) Umsetzung mindestens eines Diamins der allgemeinen Formel H₂N-A-NH₂, in der das Brückenglied A für C₁- bis C₂₀-Alkylen, welches durch bis zu 10 Sauerstoffatome und/oder tertiäre Stickstoffatome unterbrochen sein kann, C₂- bis C₂₀-Alkenylen, C₅- bis C₂₀-Cylcoalkylen, C₆- bis C₂₀-Arylen oder C₇-bis C₂₀-Aralkylen steht, mit mindestens einem C₁- bis C₁₂-Aldehyd und mindestens einem C₁- bis C₈-Alkanol bei einer Temperatur von 20 bis 80°C unter Abspaltung und Entfernung von Wasser, wobei sowohl der Aldehyd als auch der Alkohol in jeweils mehr als der doppelten molaren Menge gegenüber dem Diamin eingesetzt werden;
(B) Umsetzung des Kondensationsproduktes aus Reaktionsschritt (A) mit mindestens einem Phenol, welches mindestens einen langkettigen Substituenten mit 6 bis 3000 Kohlenstoffatomen trägt, im stöchiometrischen Verhältnis zum ursprünglich in Schritt (A) eingesetzten Diamin von 1,2 : 1 bis 3,5 : 1 bei einer Temperatur von 30 bis 120°C;
(C) Erhitzen des Umsetzungsproduktes aus Reaktionsschritt (B) auf eine Temperatur von 125 bis 280°C für mindestens 10 Minuten.

2. Polytetrahydrobenzoxazine nach Anspruch 1, erhältlich in Reaktionsschritt (A) aus mindestens einem Diamin der allgemeinen Formel H₂N-(CH₂)_{z}-NH₂, in der z für eine Zahl von 1 bis 10 steht.

3. Polytetrahydrobenzoxazine nach Anspruch 1 oder 2, erhältlich in Reaktionsschritt (A) aus Formaldehyd oder einer polymeren Form von Formaldehyd.

4. Polytetrahydrobenzoxazine nach den Ansprüchen 1 bis 3, erhältlich in Reaktionsschritt (A) aus mindestens einem C₃- oder C₄-Alkanol.

5. Polytetrahydrobenzoxazine nach den Ansprüchen 1 bis 4, wobei in Reaktionsschritt (A) das stöchiometrische Verhältnis von Diamin zu Aldehyd 1 : 4 beträgt, wobei eine Abweichung von diesem Verhältnis von bis zu 10 % toleriert werden kann, und das Alkanol in der mindestens 3,5-fachen molaren Mengen gegenüber dem Diamin eingesetzt wird.

6. Polytetrahydrobenzoxazine nach den Ansprüchen 1 bis 5, erhältlich in Reaktionsschritt (B) aus mindestens einem Phenol, welches in para-Stellung zur Hydroxylgruppe einen C₈- bis C₁₂-Alkylrest oder einen Polyisobutylrest mit 16 bis 3000 Kohlenstoffatomen trägt.

7. Polytetrahydrobenzoxazine nach den Ansprüchen 1 bis 6 mit einem zahlenmittleren Molekulargewicht (Mₙ) von 700 bis 50.000 und einem Polydispersitätsindex (PDI) von 1,5 bis 7,5.

8. Quaternisierte Polytetrahydrobenzoxazine, erhältlich durch die Reaktionsschritte (A), (B) und (C) gemäß den Ansprüchen 1 bis 7 und zusätzlich den Reaktionsschritt
(D) Quaternisierung eines Teiles oder aller quaternisierbaren Aminofunktionen des Umsetzungsproduktes aus Reaktionsschritt (C).

9. Quaternisierte Polytetrahydrobenzoxazine nach Anspruch 8, erhältlich in Reaktionsschritt (D) durch Quaternisierung mit mindestens einem Epoxid.

10. Verfahren zur Herstellung von Polytetrahydrobenzoxazinen, **dadurch gekennzeichnet, dass** man sukzessive die Reaktionsschritte
(A) Umsetzung mindestens eines Diamins der allgemeinen Formel H₂N-A-NH₂, in der das Brückenglied A für C₁- bis C₂₀-Alkylen, C₅- bis C₂₀-Cylcoalkylen, C₆- bis C₂₀-Arylen oder C₇- bis C₂₀-Aralkylen steht, mit mindestens einem C₁- bis C₁₂-Aldehyd und mindestens einem C₁- bis C₈-Alkanol bei einer Temperatur von 20 bis 80°C unter Abspaltung und Entfernung von Wasser, wobei sowohl der Aldehyd als auch der Alkohol jeweils in mehr als der doppelten molaren Menge gegenüber dem Diamin eingesetzt werden;
(B) Umsetzung des Kondensationsproduktes aus Reaktionsschritt (A) mit mindestens einem Phenol, welches mindestens einen langkettigen Substituenten mit 6 bis 3000 Kohlenstoffatomen trägt, im stöchiometrischen Verhältnis zum ursprünglich in Schritt (A) eingesetzten Alkylendiamin von 1,2 : 1 bis 3 : 1 bei einer Temperatur von 30 bis 120°C;
(C) Erhitzen des Umsetzungsproduktes aus Reaktionsschritt (B) auf eine Temperatur von 125 bis 280°C für mindestens 10 Minuten;
durchführt.

11. Verfahren zur Herstellung von quaternisierten Polytetrahydrobenzoxazinen, **dadurch gekennzeichnet, dass** man sukzessive die Reaktionsschritte (A), (B) und (C) gemäß Anspruch 10 und zusätzlich den Reaktionsschritt
(D) Quaternisierung eines Teiles oder aller quaternisierbaren Aminofunktionen des Umsetzungsproduktes aus Reaktionsschritt (C)
durchführt.

12. Polytetrahydrobenzoxazine der allgemeinen Formel I 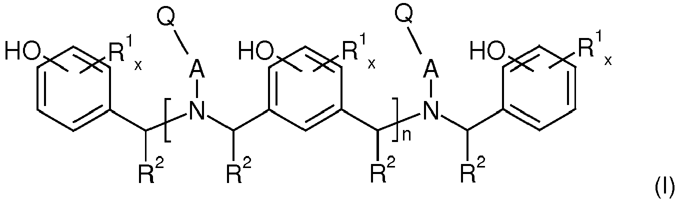 in der
x für die Zahl 1, 2, 3 oder 4 steht, wobei die Werte für x an den verschiedenen aromatischen Ringen unterschiedlich sein können,
n für eine ganze Zahl von 2 bis 10 steht,
R¹ gleiche oder verschiedene C₁- bis C₃₀₀₀-Hydrocarbylreste bezeichnet, wobei jeder Benzolkern mindestens einen C₆- bis C₃₀₀₀-Hydrocarbylrest trägt,
R² Wasserstoff oder gleiche oder verschiedene C₁- bis C₁₁-Alkylreste bezeichnet,
A ein Brückenglied mit 2 bis 20 Kohlenstoffatomen bedeutet und
Q den über ein Stickstoffatom angebundenen Rest einer Tetrahydrobenzoxazin-Einheit bezeichnet, welche in cyclischer Form gemäß der Formel 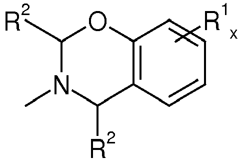 vorliegen kann, wobei die Variablen R¹, R² und x die oben genannten Bedeutungen haben.

13. Bistetrahydrobenzoxazine der allgemeinen Formel II 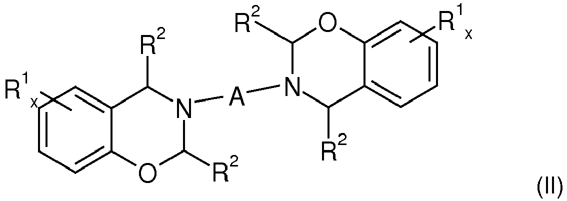 in der
x für die Zahl 1, 2, 3 oder 4 steht, wobei die Werte für x an den beiden aromatischen Ringen unterschiedlich sein können,
R¹ gleiche oder verschiedene C₁- bis C₃₀₀₀-Hydrocarbylreste bezeichnet, wobei jeder Benzolkern mindestens einen Hydrocarbylrest trägt, ausgewählt aus der Gruppe bestehend aus Polyisobutylrest mit 16 bis 3000 Kohlenstoffatomen, n-Nonyl-, Isononyl-, n-Decyl-, 2-Propylheptyl-, n-Undecyl-, n-Dodecyl-, n-Tridecyl-, Isotridecyl-, n-Tetradecyl-, n-Hexadecyl-, n-Octadecyl-, Oleyl-, Linolyl- oder Linolenylreste,
R² Wasserstoff oder gleiche oder verschiedene C₁- bis C₁₁-Alkylreste bezeichnet und
A ein Brückenglied mit 1 bis 20 Kohlenstoffatomen bedeutet.

14. Verwendung der Polytetrahydrobenzoxazine oder quaternisierten Polytetrahydrobenzoxazine oder Bistetrahydrobenzoxazine gemäß den Ansprüchen 1 bis 13 als Kraftstoffadditiv oder Schmierstoffadditiv.

15. Verwendung nach Anspruch 14 als Detergensadditiv für Dieselkraftstoffe.

16. Verwendung nach Anspruch 15 als Additiv zur Verringerung oder Vermeidung von Ablagerungen in Einspritzsystemen von direkteinspritzenden Dieselmotoren, insbesondere in Common-Rail-Einspritzsystemen, zur Verringerung des Kraftstoffverbrauches von direkteinspritzenden Dieselmotoren, insbesondere von Dieselmotoren mit Common-Rail-Einspritzsystemen, und/oder zur Minimierung des Leistungsverlustes (powerloss) in direkteinspritzenden Dieselmotoren, insbesondere in Dieselmotoren mit Common-Rail-Einspritzsystemen.

17. Additivkonzentrat, enthaltend in Kombination mit weiteren Kraftstoffadditiven, insbesondere Dieselkraftstoffadditiven, wenigstens ein Polytetrahydrobenzoxazin oder quaternisiertes Polytetrahydrobenzoxazin oder Bistetrahydrobenzoxazin gemäß den Ansprüchen 1 bis 13.

18. Kraftstoffzusammensetzung, enthaltend in einer Hauptmenge eines üblichen Grundkraftstoffes eine wirksame Menge wenigstens eines Polytetrahydrobenzoxazins oder quaternisierten Polytetrahydrobenzoxazins oder Bistetrahydrobenzoxazins gemäß den Ansprüchen 1 bis 13.

19. Schmierstoffzusammensetzung, enthaltend in einer Hauptmenge einer üblichen Schmierstoffformulierung eine wirksame Menge wenigstens eines Polytetrahydrobenzoxazins oder quaternisierten Polytetrahydrobenzoxazins oder Bistetrahydrobenzoxazins gemäß den Ansprüchen 1 bis 13.

## Claims

1. A polytetrahydrobenzoxazine obtainable by the reaction steps of:
(A) reacting at least one diamine of the general formula H₂N-A-NH₂, in which the bridging member A is C₁- to C₂₀-alkylene which may be interrupted by up to 10 oxygen atoms and/or tertiary nitrogen atoms, C₂- to C₂₀-alkenylene, C₅- to C₂₀-cycloalkylene, C₆- to C₂₀-arylene or C₇- to C₂₀-aralkylene with at least one C₁- to C₁₂-aldehyde and at least one C₁- to C₈-alkanol at a temperature of 20 to 80°C with elimination and removal of water, where both the aldehyde and the alcohol may be used in each case in more than double the molar amount compared to the diamine;
(B) reacting the condensation product from reaction step (A) with at least one phenol which bears at least one long-chain substituent having 6 to 3000 carbon atoms in a stoichiometric ratio to the diamine originally used in step (A) of 1.2 : 1 to 3.5 : 1 at a temperature of 30 to 120°C;
(C) heating the reaction product from reaction step (B) to a temperature of 125 to 280°C for at least 10 minutes.

2. A polytetrahydrobenzoxazine according to claim 1, obtainable in reaction step (A) from at least one diamine of the general formula H₂N-(CH₂)_{z}-NH₂ in which z is a number from 1 to 10.

3. A polytetrahydrobenzoxazine according to claim 1 or 2, obtainable in reaction step (A) from formaldehyde or a polymeric form of formaldehyde.

4. A polytetrahydrobenzoxazine according to claims 1 to 3, obtainable in reaction step (A) from at least one C₃- or C₄-alkanol.

5. A polytetrahydrobenzoxazine according to claims 1 to 4, wherein the stoichiometric ratio of diamine to aldehyde in reaction step (A) is 1 : 4, where a deviation from this reaction of up to 10% can be tolerated, and the alkanol is used in at least 3.5 times the molar amount compared to the diamine.

6. A polytetrahydrobenzoxazine according to claims 1 to 5, obtainable in reaction step (B) from at least one phenol which bears, in the para position to the hydroxyl group, a C₈- to C₁₂-alkyl radical or a polyisobutyl radical having 16 to 3000 carbon atoms.

7. A polytetrahydrobenzoxazine according to claims 1 to 6 having a number-average molecular weight (Mₙ) of 700 to 50 000 and a polydispersity index (PDI) of 1.5 to 7.5.

8. A quaternized polytetrahydrobenzoxazine obtainable by reaction steps (A), (B) and (C) according to claims 1 to 7 and additionally the reaction step of (D) quaternizing some or all quaternizable amino functions of the reaction product from reaction step (C).

9. A quaternerized polytetrahydrobenzoxazine according to claim 8, obtainable in reaction step (D) by quaternizing with at least one epoxide.

10. A process for preparing polytetrahydrobenzoxazines, which comprises successively performing the reaction steps of
(A) reacting at least one diamine of the general formula H₂N-A-NH₂, in which the bridging member A is C₁- to C₂-alkylene, C₅- to C₂₀-cycloalkylene, C₆- to C₂₀-arylene or C₇- to C₂₀-aralkylene with at least one C₁- to C₁₂-aldehyde and at least one C₁- to C₈-alkanol at a temperature of 20 to 80°C with elimination and removal of water, where both the aldehyde and the alcohol may be used in each case in more than double the molar amount compared to the diamine;
(B) reacting the condensation product from reaction step (A) with at least one phenol which bears at least one long-chain substituent having 6 to 3000 carbon atoms in a stoichiometric ratio to the diamine originally used in step (A) of 1.2 : 1 to 3 : 1 at a temperature of 30 to 120°C;
(C) heating the reaction product from reaction step (B) to a temperature of 125 to 280°C for at least 10 minutes.

11. A process for preparing quaternized polytetrahydrobenzoxazines, which comprises successively performing reaction steps (A), (B) and (C) according to claim 10 and additionally the reaction step of
(D) quaternizing some or all quaternizable amino functions of the reaction product from reaction step (C).

12. A polytetrahydrobenzoxazine of the general formula I 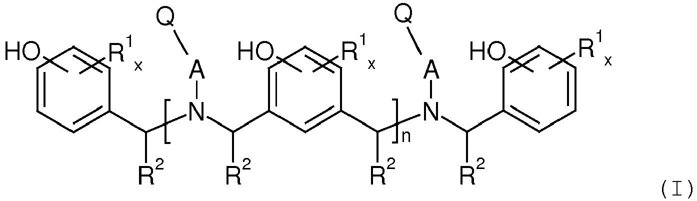 in which
x is the number 1, 2, 3 or 4, where the values of x may be different on the different aromatic rings,
n is an integer from 2 to 10,
R¹ denotes identical or different C₁- to C₃₀₀₀-hydrocarbyl radicals, where each benzene ring bears at least one C₆- to C₃₀₀₀-hydrocarbyl radical,
R² denotes hydrogen or identical or different C₁- to C₁₁-alkyl radicals,
A is a bridging member having 2 to 20 carbon atoms and
Q is the radical of a tetrahydrobenzoxazine unit which is attached via a nitrogen atom and which may be present in cyclic form according to the formula 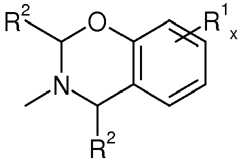 where the variables R¹, R² and x are each as defined above.

13. A bistetrahydrobenzoxazine of the general formula II 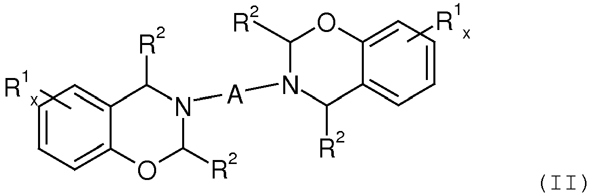 in which
x is the number 1, 2, 3 or 4, where the values of x may be different on the two different aromatic rings,
R¹ denotes identical or different C₁- to C₃₀₀₀-hydrocarbyl radicals, where each benzene ring bears at least one hydrocarbyl radical, selected from the group consisting of polyisobutyl radical having 16 to 3000 carbon atoms, n-nonyl, isononyl, n-decyl, 2-propylheptyl, n-undecyl, n-dodecyl, n-tridecyl, isotridecyl, n-tetradecyl, n-hexadecyl, n-octadecyl, oleyl, linolyl or linolenyl radicals,
R² denotes hydrogen or identical or different C₁- to C₁₁-alkyl radicals,
A is a bridging member having 1 to 20 carbon atoms.

14. The use of the polytetrahydrobenzoxazine or quaternized polytetrahydrobenzoxazines or bistetrahydrobenzoxazines according to claims 1 to 13 as a fuel additive or lubricant additive.

15. The use according to claim 14 as a detergent additive for diesel fuels.

16. The use according to claim 15 as an additive for reducing or preventing deposits in injection systems of direct-injection diesel engines, especially in common-rail injection systems, for reducing fuel consumption of direct-injection diesel engines, especially of diesel engines with common-rail injection systems, and/or for minimizing power loss in direct-injection diesel engines, especially in diesel engines with common-rail injection systems.

17. An additive concentration comprising, in combination with further fuel additives, especially diesel fuel additives, at least one polytetrahydrobenzoxazine or quaternized polytetrahydrobenzoxazine or bistetrahydrobenzoxazine according to claims 1 to 13.

18. A base fuel composition comprising, in a majority of a customary base fuel, an effective amount of at least one polytetrahydrobenzoxazine or quaternized polytetrahydrobenzoxazine or bistetrahydrobenzoxazine according to claims 1 to 13.

19. A base lubricant composition comprising, in a majority of a customary base lubricant, an effective amount of at least one polytetrahydrobenzoxazine or quaternized polytetrahydrobenzoxazine or bistetrahydrobenzoxazine according to claims 1 to 13.

## Revendications

1. Polytétrahydrobenzoxazines, pouvant être obtenues par les étapes de réaction suivantes :
(A) la mise en réaction d'au moins une diamine de formule générale H₂N-A-NH₂, dans laquelle l'élément de pont A représente alkylène en C₁ à C₂₀, qui peut être interrompu par jusqu'à 10 atomes d'oxygène et/ou atomes d'azote tertiaires, alcénylène en C₂ à C₂₀, cycloalkylène en C₅ à C₂₀, arylène en C₆ à C₂₀ ou aralkylène en C₇ à C₂₀, avec au moins un aldéhyde en C₁ à C₁₂ et au moins un alcanol en C₁ à C₈, à une température de 20 à 80 °C, avec clivage et élimination d'eau, aussi bien l'aldéhyde que l'alcool étant utilisés chacun en une quantité molaire plus que double par rapport à la diamine ;
(B) la mise en réaction du produit de condensation de l'étape de réaction (A) avec au moins un phénol, qui porte au moins un substituant à chaîne longue de 6 à 3 000 atomes de carbone, en un rapport stoechiométrique par rapport à la diamine utilisée initialement à l'étape (A) de 1,2:1 à 3,5:1, à une température de 30 à 120 °C ;
(C) le chauffage du produit de réaction de l'étape de réaction (B) à une température de 125 à 280 °C pendant au moins 10 minutes.

2. Polytétrahydrobenzoxazines selon la revendication 1, pouvant être obtenues à l'étape de réaction (A) à partir d'au moins une diamine de formule générale H₂N-(CH₂)_{z}-NH₂, dans laquelle z représente un nombre de 1 à 10.

3. Polytétrahydrobenzoxazines selon la revendication 1 ou 2, pouvant être obtenues à l'étape de réaction (A) à partir de formaldéhyde ou d'une forme polymère de formaldéhyde.

4. Polytétrahydrobenzoxazines selon les revendications 1 à 3, pouvant être obtenues à l'étape de réaction (A) à partir d'au moins un alcanol en C₃ ou C₄.

5. Polytétrahydrobenzoxazines selon les revendications 1 à 4, dans lesquelles le rapport stoechiométrique entre la diamine et l'aldéhyde à l'étape de réaction (A) est de 1:4, une déviation de ce rapport de jusqu'à 10 % pouvant être tolérée, et l'alcanol est utilisé en une quantité molaire d'au moins 3,5 fois celle de la diamine.

6. Polytétrahydrobenzoxazines selon les revendications 1 à 5, pouvant être obtenues à l'étape de réaction (B) à partir d'au moins un phénol, qui porte en position para du groupe hydroxyle un radical alkyle en C₈ à C₁₂ ou un radical polyisobutyle de 16 à 3 000 atomes de carbone.

7. Polytétrahydrobenzoxazines selon les revendications 1 à 6, ayant un poids moléculaire moyen en nombre (Mₙ) de 700 à 50 000 et un indice de polydispersité (PDI) de 1, 5 à 7,5.

8. Polytétrahydrobenzoxazines quaternisées, pouvant être obtenues par les étapes de réaction (A), (B) et (C) selon les revendications 1 à 7 et en outre l'étape de réaction
(D) la quaternisation d'une partie ou de toutes les fonctions amino quaternisables du produit de réaction de l'étape de réaction (C).

9. Polytétrahydrobenzoxazines quaternisées selon la revendication 8, pouvant être obtenues à l'étape de réaction (D) par quaternisation avec au moins un époxyde.

10. Procédé de fabrication de polytétrahydrobenzoxazines, **caractérisé en ce que** les étapes de réaction
(A) la mise en réaction d'au moins une diamine de formule générale H₂N-A-NH₂, dans laquelle l'élément de pont A représente alkylène en C₁ à C₂₀, cycloalkylène en C₅ à C₂₀, arylène en C₆ à C₂₀ ou aralkylène en C₇ à C₂₀, avec au moins un aldéhyde en C₁ à C₁₂ et au moins un alcanol en C₁ à C₈, à une température de 20 à 80 °C, avec clivage et élimination d'eau, aussi bien l'aldéhyde que l'alcool étant utilisés chacun en une quantité molaire plus que double par rapport à la diamine ;
(B) la mise en réaction du produit de condensation de l'étape de réaction (A) avec au moins un phénol, qui porte au moins un substituant à chaîne longue de 6 à 3 000 atomes de carbone, en un rapport stoechiométrique par rapport à l'alkylène-diamine utilisée initialement à l'étape (A) de 1,2:1 à 3:1, à une température de 30 à 120 °C ;
(C) le chauffage du produit de réaction de l'étape de réaction (B) à une température de 125 à 280 °C pendant au moins 10 minutes,
sont réalisées successivement.

11. Procédé de fabrication de polytétrahydrobenzoxazines quaternisées, **caractérisé en ce que** les étapes de réaction (A), (B) et (C) selon la revendication 10 et en outre l'étape de réaction
(D) la quaternisation d'une partie ou de toutes les fonctions amino quaternisables du produit de réaction de l'étape de réaction (C),
sont réalisées successivement.

12. Polytétrahydrobenzoxazines de formule générale I 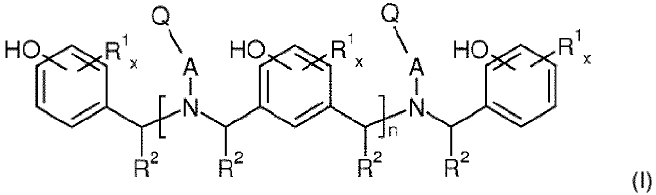 dans laquelle
x représente le nombre 1, 2, 3 ou 4, les valeurs pour x pouvant être différentes sur les différents cycles aromatiques,
n représente un nombre entier de 2 à 10,
R¹ représente des radicaux hydrocarbyle en C₁ à C₃₀₀₀ identiques ou différents, chaque noyau benzène portant au moins un radical hydrocarbyle en C₆ à C₃₀₀₀,
R² représente l'hydrogène ou des radicaux alkyle en C₁ à C₁₁ identiques ou différents,
A signifie un élément de pont de 2 à 20 atomes de carbone, et
Q représente le radical d'une unité tétrahydrobenzoxazine relié par un atome d'azote, qui peut se présenter sous forme cyclique selon la formule 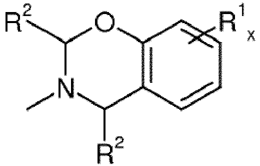 dans laquelle les variables R¹, R² et x ont les significations indiquées précédemment.

13. Bistétrahydrobenzoxazines de formule générale II 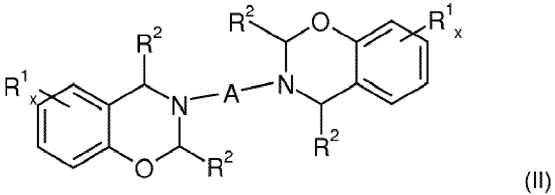 dans laquelle
x représente le nombre 1, 2, 3 ou 4, les valeurs pour x pouvant être différentes sur les deux cycles aromatiques,
R¹ représente des radicaux hydrocarbyle en C₁ à C₃₀₀₀ identiques ou différents, chaque noyau benzène portant au moins un radical hydrocarbyle, choisi dans le groupe constitué par un radical polyisobutyle de 16 à 3 000 atomes de carbone, les radicaux n-nonyle, isononyle, n-décyle, 2-propylheptyle, n-undécyle, n-dodécyle, n-tridécyle, isotridécyle, n-tétradécyle, n-hexadécyle, n-octadécyle, oléyle, linolyle ou linolényle,
R² représente l'hydrogène ou des radicaux alkyle en C₁ à C₁₁ identiques ou différents, et
A signifie un élément de pont de 1 à 20 atomes de carbone.

14. Utilisation des polytétrahydrobenzoxazines ou des polytétrahydrobenzoxazines quaternisées ou des bistétrahydrobenzoxazines selon les revendications 1 à 13 en tant qu'additif pour carburant ou additif pour lubrifiant.

15. Utilisation selon la revendication 14 en tant qu'additif détergent pour carburants diesel.

16. Utilisation selon la revendication 15 en tant qu'additif pour réduire ou éviter les dépôts dans les systèmes d'injection de moteurs diesel à injection directe, notamment dans les systèmes d'injection à rampe commune, pour réduire la consommation de carburant de moteurs diesel à injection directe, notamment de moteurs diesel à systèmes d'injection à rampe commune, et/ou pour minimiser la perte de puissance (power loss) dans des moteurs diesel à injection directe, notamment des moteurs diesel à systèmes d'injection à rampe commune.

17. Concentré d'additif, contenant en combinaison avec d'autres additifs pour carburants, notamment des additifs pour carburants diesel, au moins une polytétrahydrobenzoxazine ou une polytétrahydrobenzoxazine quaternisée ou une bistétrahydrobenzoxazine selon les revendications 1 à 13.

18. Composition de carburant, contenant dans une quantité principale d'un carburant de base usuel une quantité efficace d'au moins une polytétrahydrobenzoxazine ou une polytétrahydrobenzoxazine quaternisée ou une bistétrahydrobenzoxazine selon les revendications 1 à 13.

19. Composition de lubrifiant, contenant dans une quantité principale d'une formulation lubrifiante usuelle une quantité efficace d'au moins une polytétrahydrobenzoxazine ou une polytétrahydrobenzoxazine quaternisée ou une bistétrahydrobenzoxazine selon les revendications 1 à 13.
